# EUROPEAN PATENT APPLICATION

(11) **EP 3 330 282 A1**
(43) Date of publication of application: **06.06.2018**
(21) Application number: 16202046.5
(22) Date of filing: 02.12.2016
(51) Int. Cl.: C07K 14/24, C12R 1/01

(54) **CIPA, CIPB AND PIXA AS SCAFFOLDS TO ORGANIZE PROTEINS INTO CRYSTALLINE INCLUSIONS**

(71) Applicant: Ludwig-Maximilians-Universität München, 80539 München (DE)
(72) Inventor: Jung, Kirsten, 82152 Krailing (DE); Heermann, Ralf, 81241 München (DE); Wang, Yang, 251714 Huimin (CN)
(74) Representative: Vossius & Partner Patentanwälte Rechtsanwälte mbB

(57) **Abstract**

The present invention relates to a method for producing at least two proteins in the form of protein crystalline inclusions (PCIs) in a bacterial host cell, wherein the method comprises (a) introducing one or more fusion constructs into the bacterial host cell, said one or more fusion constructs comprising in expressible form one or more nucleic acid molecules encoding the at least two proteins, wherein each of the one or more nucleic acid molecules is independently fused to a nucleic acid molecule being selected from the group consisting of the nucleic acid molecules having i. the nucleotide sequence of SEQ ID NO: 1, ii. the nucleotide sequence of SEQ ID NO: 2, iii. the nucleotide sequence of SEQ ID NO: 3, iv. a nucleotide sequence being at least 80% identical to SEQ ID NO: 1, wherein the encoded protein retains the capability of forming PCIs, v. a nucleotide sequence being at least 80% identical to SEQ ID NO: 2, wherein the encoded protein retains the capability of forming PCIs, vi. a nucleotide sequence being at least 80% identical to SEQ ID NO: 3, wherein the encoded protein retains the capability of forming PCIs, vii. a nucleotide sequence encoding the amino acid sequence of SEQ ID NO: 4, viii. a nucleotide sequence encoding the amino acid sequence of SEQ ID NO: 5, ix. a nucleotide sequence encoding the amino acid sequence of SEQ ID NO: 6, x. a nucleotide sequence encoding an amino acid sequence being at least 80% identical to SEQ ID NO: 4, wherein the encoded protein retains the capability of forming PCIs, xi. a nucleotide sequence encoding an amino acid sequence being at least 80% identical to SEQ ID NO: 5, wherein the encoded protein retains the capability of forming PCIs, and xii. a nucleotide sequence encoding an amino acid sequence being at least 80% identical to SEQ ID NO: 6, wherein the encoded protein retains the capability of forming PCIs; and (b) culturing the bacterial host cell obtained in (a) to produce the at least two proteins in the form of PCIs.

SEQUENCE LISTING

<110> JUNG, Kirsten

<120> CipA, CipB and PixA as scaffolds to organize proteins into crystalline
inclusions

<130> Z2605 EP

<160> 54

<170> BiSSAP 1.3

<210> 1
<211> 315
<212> DNA
<213> Photorhabdus luminescens

<220>
<223> cipA nucleotide sequence

<400> 1
atgattaatg acatgcatcc ttctttaatt aaagataaag atatagtaga tgacgtaatg 60

ctacgtagct gtaaaatcat tgcaatgaaa gttatgccag acaaagttat gcaagttatg 120

gtgactgtat taatgcatga tggcgtatgt gaagaaatgc ttttgaaatg gaatctgcta 180

gacaatagag gcatggcgat ttataaagtt ctgatggaag cgctttgcgc taagaaagat 240

gtgaaaatta gcaccgtagg aaaagtaggt cctctcggct gcgattacat taattgcgta 300

gaaatctcta tgtaa 315

<210> 2
<211> 303
<212> DNA
<213> Photorhabdus luminescens

<220>
<223> cipB nucleotide sequence

<400> 2
atgataatta agaaagatat tttgttaaat gaagagctga ttgtcgatga tgatcttaaa 60

gtgggtaaag tagagaaggt caatattgat attctgtcac ccagctcagt cattgttagt 120

ctgaatattt taggcgtagt tgatgatttt catctattac tagttgatga taaagataaa 180

gataagattg tgctgctcta tctttctctt ttacgtgttc ttcatgaaaa attagatgta 240

aaagtaaaag tcgcaaaaag taaacttact aagataaaat atatagtagg tgttgaaatt 300

taa 303

<210> 3
<211> 558
<212> DNA
<213> Xenorhabdus nematophila

<220>

<223> pixA nucleotide sequence

<400> 3
atgagaaata tcgatattat gttagcagta aaagctgaag aaatcatgaa tgactatggt 60

aaactcagca aagatattaa taagcctgtt ttaattactc cctcaatact taacaaaaat 120

tatctccgtt tgttacctga agatgacagt gttgtttgtg tagatgaaga gcttactctc 180

tctattaaag ctaatatagg tgacatgatc cgtttgaatg tcagatcatt aaatattgaa 240

acaacatact ctgctgcttt ggtcaaaatt gaaccgatga atgtaatgag agatggtgtt 300

gtttcaatgg tcagccttcc tgtcgtagaa agcgttgtga gatctgttgc taccgtcgat 360

atgaaaaaca cggttgacgt agatatcaat acaatgaaag attatcactg gatgattaaa 420

gtgaacgatc tgccgagtgt agacagaatt agcacaatgt actatctcag cactatcgca 480

atttatagag ataaaatgct aatgggttat atcgcattgg aatccggcct tattctggat 540

cacacagtta tgatgtaa 558

<210> 4
<211> 104
<212> PRT
<213> Photorhabdus luminescens

<220>
<223> CipA protein sequence

<400> 4
Met Ile Asn Asp Met His Pro Ser Leu Ile Lys Asp Lys Asp Ile Val
1               5                   10                  15
Asp Asp Val Met Leu Arg Ser Cys Lys Ile Ile Ala Met Lys Val Met
            20                  25                  30
Pro Asp Lys Val Met Gln Val Met Val Thr Val Leu Met His Asp Gly
        35                  40                  45
Val Cys Glu Glu Met Leu Leu Lys Trp Asn Leu Leu Asp Asn Arg Gly
    50                  55                  60
Met Ala Ile Tyr Lys Val Leu Met Glu Ala Leu Cys Ala Lys Lys Asp
65                  70                  75                  80
Val Lys Ile Ser Thr Val Gly Lys Val Gly Pro Leu Gly Cys Asp Tyr
                85                  90                  95
Ile Asn Cys Val Glu Ile Ser Met
            100

<210> 5
<211> 100
<212> PRT
<213> Photorhabdus luminescens

<220>
<223> CipB protein sequence

<400> 5
Met Ile Ile Lys Lys Asp Ile Leu Leu Asn Glu Glu Leu Ile Val Asp
1               5                   10                  15
Asp Asp Leu Lys Val Gly Lys Val Glu Lys Val Asn Ile Asp Ile Leu
            20                  25                  30
Ser Pro Ser Ser Val Ile Val Ser Leu Asn Ile Leu Gly Val Val Asp
        35                  40                  45

Asp Phe His Leu Leu Leu Val Asp Asp Lys Asp Lys Asp Lys Ile Val
    50                  55                  60
Leu Leu Tyr Leu Ser Leu Leu Arg Val Leu His Glu Lys Leu Asp Val
65                  70                  75                  80
Lys Val Lys Val Ala Lys Ser Lys Leu Thr Lys Ile Lys Tyr Ile Val
                85                  90                  95
Gly Val Glu Ile
            100

<210> 6
<211> 185
<212> PRT
<213> Xenorhabdus nematophila

<220>
<223> PixA protein sequence

<400> 6
Met Arg Asn Ile Asp Ile Met Leu Ala Val Lys Ala Glu Glu Ile Met
1               5                   10                  15
Asn Asp Tyr Gly Lys Leu Ser Lys Asp Ile Asn Lys Pro Val Leu Ile
            20                  25                  30
Thr Pro Ser Ile Leu Asn Lys Asn Tyr Leu Arg Leu Leu Pro Glu Asp
        35                  40                  45
Asp Ser Val Val Cys Val Asp Glu Glu Leu Thr Leu Ser Ile Lys Ala
    50                  55                  60
Asn Ile Gly Asp Met Ile Arg Leu Asn Val Arg Ser Leu Asn Ile Glu
65                  70                  75                  80
Thr Thr Tyr Ser Ala Ala Leu Val Lys Ile Glu Pro Met Asn Val Met
                85                  90                  95
Arg Asp Gly Val Val Ser Met Val Ser Leu Pro Val Val Glu Ser Val
            100                 105                 110
Val Arg Ser Val Ala Thr Val Asp Met Lys Asn Thr Val Asp Val Asp
        115                 120                 125
Ile Asn Thr Met Lys Asp Tyr His Trp Met Ile Lys Val Asn Asp Leu
    130                 135                 140
Pro Ser Val Asp Arg Ile Ser Thr Met Tyr Tyr Leu Ser Thr Ile Ala
145                 150                 155                 160
Ile Tyr Arg Asp Lys Met Leu Met Gly Tyr Ile Ala Leu Glu Ser Gly
                165                 170                 175
Leu Ile Leu Asp His Thr Val Met Met
            180                 185

<210> 7
<211> 42
<212> DNA
<213> Artificial Sequence

<220>
<223> cipA-RBS-NheI-sen

<400> 7
cggctagcag gaggtctata tgattaatga catgcatcct tc                          42


<210> 8
<211> 42
<212> DNA
<213> Artificial Sequence

<220>
<223> cipB-RBS-NheI-sen

<400> 8
ccggctagca ggaggtctat atgataatta agaaagatat tt                42

<210> 9
<211> 43
<212> DNA
<213> Artificial Sequence

<220>
<223> cipA-RBS-KpnI-sen

<400> 9
cagggtacca ggaggtctat atgattaatg acatgcatcc ttc               43

<210> 10
<211> 42
<212> DNA
<213> Artificial Sequence

<220>
<223> cipB-RBS-KpnI-sen

<400> 10
cagggtacca ggaggtctat atgataatta agaaagatat tt                42

<210> 11
<211> 43
<212> DNA
<213> Artificial Sequence

<220>
<223> cipA-RBS-SalI-sen

<400> 11
cacgtcgaca ggaggtctat atgattaatg acatgcatcc ttc               43

<210> 12
<211> 42
<212> DNA
<213> Artificial Sequence

<220>
<223> cipB-RBS-SalI-sen

<400> 12
cacgtcgaca ggaggtctat atgataatta agaaagatat tt                42

<210> 13
<211> 43
<212> DNA
<213> Artificial Sequence

<220>
<223> cipA-RBS-HindIII-sen

<400> 13
gcgaagctta ggaggtctat atgattaatg acatgcatcc ttc               43

<210> 14
<211> 42
<212> DNA
<213> Artificial Sequence

<220>
<223> cipB-RBS-HindIII-sen

<400> 14
gcgaagctta ggaggtctat atgataatta agaaagatat tt                          42


<210> 15
<211> 23
<212> DNA
<213> Artificial Sequence

<220>
<223> cipA-anti

<400> 15
catagagatt tctacgcaat taa                                               23


<210> 16
<211> 23
<212> DNA
<213> Artificial Sequence

<220>
<223> cipB-anti

<400> 16
aatttcaaca cctactatat att                                               23


<210> 17
<211> 50
<212> DNA
<213> Artificial Sequence

<220>
<223> cipA-6his-HindIII-anti

<400> 17
gctaagctta gtgatggtga tggtgatgca tagagatttc tacgcaatta                  50


<210> 18
<211> 50
<212> DNA
<213> Artificial Sequence

<220>
<223> cipB-6his-HindIII-anti

<400> 18
gctaagctta gtgatggtga tggtgatgaa tttcaacacc tactatatat                  50


<210> 19
<211> 22

<212> DNA
<213> Artificial Sequence

<220>
<223> gfp-sense

<400> 19
atgagtaaag gagaagaact tt                                                22


<210> 20
<211> 29
<212> DNA
<213> Artificial Sequence

<220>
<223> gfp-HindIII-anti

<400> 20
ctaagcttat ttgtatagtt catccatgc                                         29


<210> 21
<211> 44
<212> DNA
<213> Artificial Sequence

<220>
<223> cipA-gfp-anti

<400> 21
aaagttcttc tcctttactc atcatagaga tttctacgca atta                        44


<210> 22
<211> 44
<212> DNA
<213> Artificial Sequence

<220>
<223> cipB-gfp-anti

<400> 22
aaagttcttc tcctttactc ataatttcaa cacctactat atat                        44


<210> 23
<211> 32
<212> DNA
<213> Artificial Sequence

<220>
<223> gfp-XmaI-anti

<400> 23
actcccgggt tatttgtata gttcatccat gc                                     32


<210> 24
<211> 45
<212> DNA
<213> Artificial Sequence

<220>
<223> gfp-RBS-EcoR1-sense

<400> 24
cgcgaattca ggaggatact aatgagtaaa ggagaagaac ttttc                45

<210> 25
<211> 47
<212> DNA
<213> Artificial Sequence

<220>
<223> cipA-lacZ-anti

<400> 25
cagtgaatcc gtaatcatgg tcatcataga gatttctacg caattaa              47

<210> 26
<211> 47
<212> DNA
<213> Artificial Sequence

<220>
<223> cipB-lacZ-anti

<400> 26
cagtgaatcc gtaatcatgg tcataatttc aacacctact atatatt              47

<210> 27
<211> 22
<212> DNA
<213> Artificial Sequence

<220>
<223> lacZ-Sense

<400> 27
atgaccatga ttacggattc ac                                         22

<210> 28
<211> 30
<212> DNA
<213> Artificial Sequence

<220>
<223> lacZ-HindIII-anti

<400> 28
cgcaagctta tttttgacac cagaccaact                                 30

<210> 29
<211> 48
<212> DNA
<213> Artificial Sequence

<220>
<223> cipA-vioA-sen

<400> 29
attaattgcg tagaaatctc tatgatgaag cattcttccg atatctgc                48

<210> 30
<211> 29
<212> DNA
<213> Artificial Sequence

<220>
<223> vioA-SacI-anti

<400> 30
gtcgagctca cgcggcgatg cgctgcagc                                     29

<210> 31
<211> 48
<212> DNA
<213> Artificial Sequence

<220>
<223> cipA-vioB-sen

<400> 31
attaattgcg tagaaatctc tatgatgagc attctggatt ttccacgc                48

<210> 32
<211> 33
<212> DNA
<213> Artificial Sequence

<220>
<223> vioB-KpnI-anti

<400> 32
ctgggtacct caggcctctc tagaaagctt tcc                                33

<210> 33
<211> 49
<212> DNA
<213> Artificial Sequence

<220>
<223> cipA-vioC-sen

<400> 33
attaattgcg tagaaatctc tatgatgaaa agagcaatca tagtcggag               49

<210> 34
<211> 33
<212> DNA
<213> Artificial Sequence

<220>
<223> vioC-SalI-anti

<400> 34
cctgtcgact cagttgaccc tccctatctt gta                                33

<210> 35
<211> 49
<212> DNA
<213> Artificial Sequence

<220>
<223> cipA-vioD-sen

<400> 35
attaattgcg tagaaatctc tatgatgaag attctggtca tcggcgcgg                49


<210> 36
<211> 31
<212> DNA
<213> Artificial Sequence

<220>
<223> vioD-HindIII-anti

<400> 36
cgcaagcttc agcgttgcag cgcgtagcgc a                                   31


<210> 37
<211> 48
<212> DNA
<213> Artificial Sequence

<220>
<223> cipA-vioE-sen

<400> 37
attaattgcg tagaaatctc tatgatggaa aaccgggaac cgccgctg                 48


<210> 38
<211> 33
<212> DNA
<213> Artificial Sequence

<220>
<223> vioE-HindIII-anti

<400> 38
ctcaagcttc tagcgcttgg cggcgaagac ggc                                 33


<210> 39
<211> 44
<212> DNA
<213> Artificial Sequence

<220>
<223> vioA-RBS-NheI-sen

<400> 39
ccggctagca ggaggtctat atgaagcatt cttccgatat ctgc                     44


<210> 40
<211> 44

<212> DNA
<213> Artificial Sequence

<220>
<223> vioB-RBS-KpnI-sen

<400> 40
cagggtacca ggaggtctat atgagcattc tggattttcc acgc                44


<210> 41
<211> 45
<212> DNA
<213> Artificial Sequence

<220>
<223> vioC-RBS-KpnI-sen

<400> 41
cagggtacca ggaggtctat atgaaaagag caatcatagt cggag               45


<210> 42
<211> 45
<212> DNA
<213> Artificial Sequence

<220>
<223> vioD-RBS-SalI-sen

<400> 42
cacgtcgaca ggaggtctat atgaagattc tggtcatcgg cgcgg               45


<210> 43
<211> 44
<212> DNA
<213> Artificial Sequence

<220>
<223> vioE-RBS-HindIII-sen

<400> 43
gcgaagctta ggaggtctat atggaaaacc gggaaccgcc gctg                44


<210> 44
<211> 29
<212> DNA
<213> Artificial Sequence

<220>
<223> Del-cipA-upstream-PstI-sen

<400> 44
ctctgcagca tagaatagtg aattctaat                                 29


<210> 45
<211> 46
<212> DNA
<213> Artificial Sequence

<220>
<223> Del-cipA-upstream-up-anti

<400> 45
aagcatttca acaatcaatt attaatgtaa actccaaaat ataatg                    46


<210> 46
<211> 21
<212> DNA
<213> Artificial Sequence

<220>
<223> Del-cipA-downstream-sen

<400> 46
aataattgat tgttgaaatg c                                               21


<210> 47
<211> 29
<212> DNA
<213> Artificial Sequence

<220>
<223> Del-cipA-downsteam-BamHI-anti

<400> 47
gcggatccta tatgactgca atctctgaa                                       29


<210> 48
<211> 30
<212> DNA
<213> Artificial Sequence

<220>
<223> Del-cipB-upstream-PstI-sen

<400> 48
attctgcagg tggaagtgaa ggggcagtgg                                      30


<210> 49
<211> 42
<212> DNA
<213> Artificial Sequence

<220>
<223> Del-cipB-upstream-up-anti

<400> 49
tagaaattta acaaatactt cgacataaac tccaaaattt aa                        42


<210> 50
<211> 20
<212> DNA
<213> Artificial Sequence

<220>
<223> Del-cipB-downstream-sen

<400> 50
gaagtatttg ttaaatttct                                                    20

<210> 51
<211> 24
<212> DNA
<213> Artificial Sequence

<220>
<223> Del-cipB-downsteam-NheI-anti

<400> 51
ataacgggaa cgccatgcta gcgg                                               24

<210> 52
<211> 32
<212> DNA
<213> Artificial Sequence

<220>
<223> Promoter-cipA-PstI-sens

<400> 52
ggcctgcagt tactccaata caatttatcc tt                                      32

<210> 53
<211> 31
<212> DNA
<213> Artificial Sequence

<220>
<223> Promoter-cipB-PstI-sens

<400> 53
ggcctgcagc gtggtatagt taaaattgac a                                       31

<210> 54
<211> 37
<212> DNA
<213> Artificial Sequence

<220>
<223> gfp-EagI-anti

<400> 54
ctaagcttcg gccgttattt gtatagttca tccatgc                                 37

## Description

The present invention relates to a method for producing at least two proteins in the form of protein crystalline inclusions (PCIs) in a bacterial host cell, wherein the method comprises (a) introducing one or more fusion constructs into the bacterial host cell, said one or more fusion constructs comprising in expressible form one or more nucleic acid molecules encoding the at least two proteins, wherein each of the one or more nucleic acid molecules is independently fused to a nucleic acid molecule being selected from the group consisting of the nucleic acid molecules having i. the nucleotide sequence of SEQ ID NO: 1, ii. the nucleotide sequence of SEQ ID NO: 2, iii. the nucleotide sequence of SEQ ID NO: 3, iv. a nucleotide sequence being at least 80% identical to SEQ ID NO: 1, wherein the encoded protein retains the capability of forming PCIs, v. a nucleotide sequence being at least 80% identical to SEQ ID NO: 2, wherein the encoded protein retains the capability of forming PCIs, vi. a nucleotide sequence being at least 80% identical to SEQ ID NO: 3, wherein the encoded protein retains the capability of forming PCls, vii. a nucleotide sequence encoding the amino acid sequence of SEQ ID NO: 4, viii. a nucleotide sequence encoding the amino acid sequence of SEQ ID NO: 5, ix. a nucleotide sequence encoding the amino acid sequence of SEQ ID NO: 6, x. a nucleotide sequence encoding an amino acid sequence being at least 80% identical to SEQ ID NO: 4, wherein the encoded protein retains the capability of forming PCIs, xi. a nucleotide sequence encoding an amino acid sequence being at least 80% identical to SEQ ID NO: 5, wherein the encoded protein retains the capability of forming PCls, and xii. a nucleotide sequence encoding an amino acid sequence being at least 80% identical to SEQ ID NO: 6, wherein the encoded protein retains the capability of forming PCIs,; and (b) culturing the bacterial host cell obtained in (a) to produce the at least two proteins in the form of PCIs.

In this specification, a number of documents including patent applications and manufacturer's manuals are cited. The disclosure of these documents, while not considered relevant for the patentability of this invention, is herewith incorporated by reference in its entirety. More specifically, all referenced documents are incorporated by reference to the same extent as if each individual document was specifically and individually indicated to be incorporated by reference.

Organized protein complexes are ubiquitous in bacteria, including such diverse functional macromolecular assemblies as ribosomes or photosystems. *^{1,2}* At the subcellular level, proteins can be organized into compartments such as carboxysomes *^{3, 4}* and gas vesicles*⁵*, or into protein inclusions like the parasporal crystalline inclusion (Bt crystals)*^{6, 7}* or refractile body (R body).*^{8, 9}* Based on the principles of natural protein organization, various types of synthetic protein scaffolds have been developed to artificially organize proteins with a view to promoting *in vivo* or *in vitro* biotechnological processes*^{10, 11}* or mimicking the function of organelles.*^{12, 13}* For example, to fine-tune metabolic flux through mevalonate and glucaric acid biosynthetic pathways, the related enzymes have been spatially organized into supramolecules via the scaffolding of signaling protein interaction domains.*¹⁴* At subcellular scales, a lipid-containing scaffold has recently been developed that can co-organize two enzymes involved in indigo biosynthesis into lipid-bound compartments in *Escherichia coli* for the purpose of metabolic engineering. *¹⁵* The cohesin-dockerin scaffold has been used to display multiple enzymes on bacterial outer membrane vesicles for *in-vitro* cascade reactions.*¹⁶* An ethanol bioreactor was generated by incorporating the corresponding enzymes into an intracellular compartment normally devoted to 1,2-propanediol utilization in *Citrobacter freundii*.*¹⁷* Moreover, a method for scaffold-free self-assembly of the octameric leucine dehydrogenase and dimeric formate dehydrogenase has been developed.*¹⁸*

The present invention aims at providing novel scaffolds that can organize proteins into specific cellular compartments and that can be used in methods for producing one or more proteins being organized into these cellular compartments.

Hence, the present invention relates in a first aspect to a method for producing at least two proteins in the form of protein crystalline inclusions (PCIs) in a bacterial host cell, wherein the method comprises (a) introducing one or more fusion constructs into the bacterial host cell, said one or more fusion constructs comprising in expressible form one or more nucleic acid molecules encoding the at least two proteins, wherein each of the one or more nucleic acid molecules is independently fused to a nucleic acid molecule being selected from the group consisting of the nucleic acid molecules having i. the nucleotide sequence of SEQ ID NO: 1, ii. the nucleotide sequence of SEQ ID NO: 2, iii. the nucleotide sequence of SEQ ID NO: 3, iv. a nucleotide sequence being at least 80% identical to SEQ ID NO: 1, wherein the encoded protein retains the capability of forming PCls, v. a nucleotide sequence being at least 80% identical to SEQ ID NO: 2, wherein the encoded protein retains the capability of forming PCls, vi. a nucleotide sequence being at least 80% identical to SEQ ID NO: 3, wherein the encoded protein retains the capability of forming PCIs, vii. a nucleotide sequence encoding the amino acid sequence of SEQ ID NO: 4, viii. a nucleotide sequence encoding the amino acid sequence of SEQ ID NO: 5, ix. a nucleotide sequence encoding the amino acid sequence of SEQ ID NO: 6, x. a nucleotide sequence encoding an amino acid sequence being at least 80% identical to SEQ ID NO: 4, wherein the encoded protein retains the capability of forming PCls, xi. a nucleotide sequence encoding an amino acid sequence being at least 80% identical to SEQ ID NO: 5, wherein the encoded protein retains the capability of forming PCls, and xii. a nucleotide sequence encoding an amino acid sequence being at least 80% identical to SEQ ID NO: 6, wherein the encoded protein retains the capability of forming PCIs,; and (b) culturing the bacterial host cell obtained in (a) to produce the at least two proteins in the form of PCIs.

The term "protein" as used herein is not limited to any particular protein. It includes proteins having an enzymatic activity, for example enzymes such as kinases, proteases, phosphatases, oxidases and reductases as well as non-enzyme proteins, for example proteins comprising immunoglobulin domains such as soluble antibodies. Membrane proteins, comprising peripheral membrane proteins and integral membrane proteins are also included. Examples of membrane proteins include receptors. Receptors include growth factor receptors, G-protein coupled receptors and receptors with seven, but also with less or more than seven transmembrane helices. The term "protein" refers to single-domain proteins as well as to multi-domain proteins. Further embraced are embodiments, wherein the protein functions as part of a multi-step biosynthetic pathway. The protein may consist of or comprise protein domains known to function in signal transduction and/or known to be involved in protein-protein interactions. Further information about these and other protein domains is available from the databases InterPro (http://www.ebi.ac.uk/interpro/, Mulder et al., 2003), Pfam (http://www.sanger.ac.uk/Software/Pfam/, Bateman et al., 2004) and SMART (http://smart.emblheidelberg.de/, Letunic et al., 2004). The term "protein" also encompasses parts of mature proteins, such as certain protein domains or only those parts of a protein conferring a desired activity, such as the enzymatic activity of an enzyme.

Each protein to be produced in the form of PCIs may be a heterologous protein or a homologous protein and is preferably a heterologous protein. A heterologous protein refers to a protein being comprised or produced in a host cell, which host cell does not naturally comprise and produce this protein. An example is the recombinant production of a human protein in an *Escherichia coli* host cell. A homologous protein refers to a protein being comprised or produced in a host cell, which naturally comprises and produces this protein. An example is the recombinant production of an *Escherichia coli* protein in an *Escherichia coli* host cell. It is to be understood that the protein to be produced in the form of PCIs is not itself a PCI-forming protein, such as CipA, CipB or PixA or a derivative thereof being capable of forming PCIs.

*Photorhabdus spec.* and *Xenorhabdus spec.* are two genera of Gram-negative insect pathogenic bacteria (family Enterobacteriaceae) found in symbiotic association with entomopathogenic nematodes of the families Heterorhabditidae and Steinernematidae, respectively (see You et al. (2006), FEMS Microbiol Ecol.; 55(2):178-85). Two types of protein crystalline inclusions are found in the cytoplasm of *Xenorhabdus spec.* and *Photorhabdus spec.* cells. Consequently they are referred to herein as protein crystalline inclusions (PCIs). The genes encoding these crystal proteins in *Photorhabdus* cells have been identified and sequenced. The genes have been named *cipA* (SEQ ID NO: 1) and *cipB* (SEQ ID NO: 2). Moreover, one of the genes encoding these crystal proteins in *Xenorhabdus spec.* cells has been identified and sequenced (see Goetsch et al. (2006), J Bacteriol., 188(7): 2706-2710). This isolated gene has been named *pixA* (SEQ ID NO: 3).

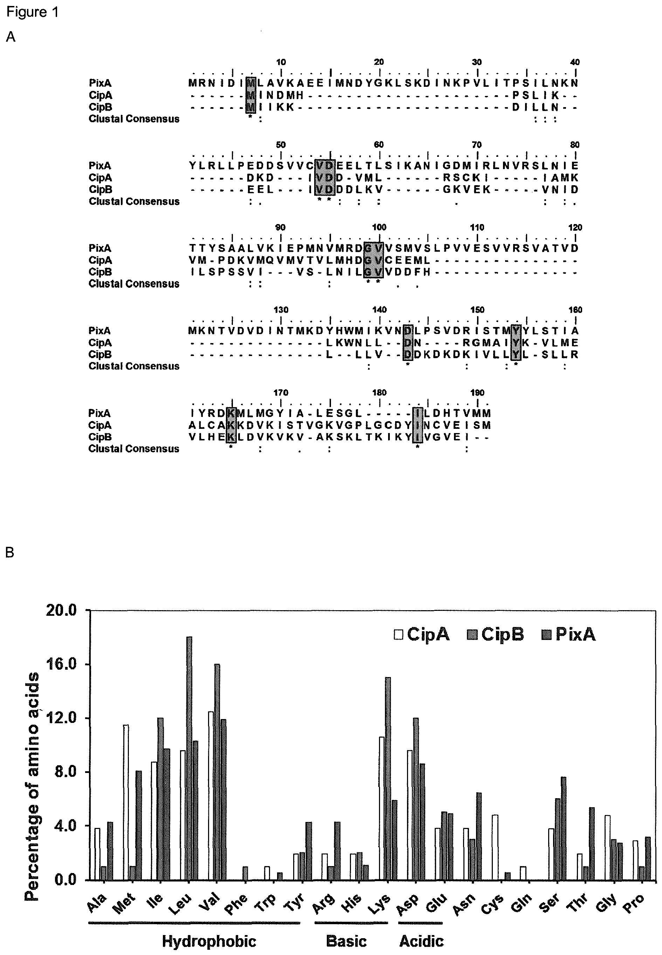

The corresponding proteins are termed CipA (SEQ ID NO: 4), CipB (SEQ ID NO: 5) and PixA (SEQ ID NO: 6). CipA and CipB are small proteins of 104 and 100 amino acids respectively, which are found in the entomopathogenic bacterium *Photorhabdus luminescens* (Figure 1A). PixA as found in *Xenorhabdus spec.* is a protein of 185 amino acids. CipA and CipB PCIs appear refractile when viewed by phase-contrast microscopy, and exhibit a polyhedral configuration in the scanning or transmission electron microscope.*^{19, 20}* The PCIs are soluble in high concentrations of urea or at alkaline pH.*²⁰* The natural function of the PCIs is still unclear*¹⁹⁻²²*. CipA, CipB and PixA display no significant sequence similarity to each other or to other proteins, and assemble independently into PCIs.*^{19, 20}* However and as shown in Figure 1B the amino acid composition of CipA, CipB and PixA is very similar. All three proteins are characterized by high proportions of hydrophobic amino acids. CipA and CipB comprise, respectively, 49.0% and 51.0% hydrophobic amino acids. Also PixA comprises 49.1% hydrophobic amino acids. It is believed that their hydrophobicity is the primary driving force of the PCI formation.

As shown in the examples herein below the PCIs cannot only be formed in their natural environment in *Xenorhabdus spec.* and *Photorhabdus spec.* host cells but surprisingly also upon the heterologous expression of the *cipA* or *cipB* gene in *Escherichia coli* host cells. For this reason the bacterial host cell to be used in connection with the invention is not particularly limited. The bacterial host cell is preferably a eubacterial host cell and more preferably a Gram-negative bacterial host cell. Further suitable host cells will be detailed herein below.

Also encompassed by the embodiments of the present invention are sequences being at least 80% identical to one or more of SEQ ID NOs 1 to 6. In accordance with the present invention, the term "percent (%) sequence identity" describes the number of matches ("hits") of identical nucleotides/amino acids of two or more aligned nucleic acid or amino acid sequences as compared to the number of nucleotides or amino acid residues making up the overall length of the template nucleic acid or amino acid sequences. In other terms, using an alignment, for two or more sequences or subsequences the percentage of amino acid residues or nucleotides that are the same (e.g. 80%, 85%, 90% or 95% identity) may be determined, when the (sub)sequences are compared and aligned for maximum correspondence over a window of comparison, or over a designated region as measured using a sequence comparison algorithm as known in the art, or when manually aligned and visually inspected. The sequences which are compared to determine sequence identity may thus differ by substitution(s), addition(s) or deletion(s) of nucleotides or amino acids. This definition also applies to the complement of a test sequence.

The skilled person is also aware of suitable programs to align nucleic acid sequences. The percentage sequence identity of polypeptide sequences can, for example, be determined with programmes as the above explained programmes CLUSTLAW, FASTA and BLAST. Preferably the BLAST programme is used, namely the NCBI BLAST algorithm (Stephen F. Altschul, Thomas L. Madden, Alejandro A. Schäffer, Jinghui Zhang, Zheng Zhang, Webb Miller, and David J. Lipman (1997), "Gapped BLAST and PSI-BLAST: a new generation of protein database search programs", Nucleic Acids Res. 25:3389-3402).

With regard to the sequence identity as recited herein in items (iv) to (vi) and (x) to (xii), it is preferred with increasing preference that the sequence identity is at least 85%, at least 90%, at least 95%, at least 98% and at least 99%. With regard to the nucleic acid molecules of items (iv) to (vi) and (x) to (xii) and preferred embodiments thereof, it is to be understood that these nucleic acid molecules encode proteins retain the capability of forming PCIs of CipA, CipB and PixA.

It is furthermore preferred with respect to the nucleotide sequences being at least 80% identical to SEQ ID NOs 1 and 3 that at least 35% and preferably at least 45% of the nucleotide triplets comprised therein encode hydrophobic amino acids. Similarly, with respect to the nucleotide sequence being at least 80% identical to SEQ ID NO: 2 it is preferred that at least 40% and preferably at least 50% of the nucleotide triplets comprised therein encode hydrophobic amino acids. It is preferred with respect to the nucleotide sequences being at least 80% identical to SEQ ID NOs 1 to 3 that no nucleotides are deleted, replaced or added in nucleotide triplets encoding the hydrophobic amino acids of SEQ ID NOs 1 and 3, respectively. Alternatively, they are replaced by triplets encoding different hydrophobic amino acids. It is likewise preferred with respect to the amino acid sequences being at least 80% identical to SEQ ID NOs 4 and 6 that at least 35% and preferably at least 45% of the amino acids are hydrophobic amino acids. Similarly, with respect to the amino acid sequence being at least 80% identical to SEQ ID NO: 5 it is preferred that at least 40% and preferably at least 50% of the amino acids are hydrophobic amino acids. It is preferred with respect to the amino acid sequences being at least 80% identical to SEQ ID NOs 4 and 6 that no hydrophobic amino acids of SEQ ID NOs 4 and 6, respectively are deleted, replaced or added. Alternatively, they are replaced by triplets encoding different hydrophobic amino acids. The hydrophobic amino acids encoded by SEQ ID NOs 1 to 3 and comprised in SEQ ID NOs 4 to 6 are Ala, Ile, Leu, Val, Met, Phe, Trp and Tyr.

The term "fusion construct" as used herein comprises in expressible form one or more nucleic acid molecules encoding at least two proteins to be produced in the form of PCls, wherein each of the one or more nucleic acid molecules is independently fused to a nucleic acid molecule being selected from the group consisting of the nucleic acid molecules as referred to herein in items (i) to (xii). The term "expressible form" requires that the fusion construct harbours the nucleic acid molecule encoding the at least one protein to be produced and the protein being encoded the nucleic acid molecules as selected from items (i) to (xii) in a form that is transcribed into RNA and translated into protein in the bacterial host cell.

In its broadest from, being designated option (I), the fusion construct comprises one nucleic acid molecule encoding one protein to be produced in the form of PCIs being fused to a nucleic acid molecule being selected from the group consisting of the nucleic acid molecules as referred to herein in items (i) to (xii). In this case the fusion construct encodes a fusion protein, wherein the protein to be produced in the form of PCIs is fused to the protein encoded by the selected nucleic acid molecule as referred to herein in items (i) to (xii), noting that the protein encoded by the selected nucleic acid molecule as referred to herein in items (i) to (xii) is a PCI-forming protein. Such a fusion construct is illustrated by the vectors pBAD-cipA-gfp, pBAD-cipB-gfp, pBAD-cipA-lacZ or pBAD-cipB-lacZ as provided in the examples herein below. Hence, it is be understood that the fusion construct pursuant to option (I) encodes only one fusion protein, said fusion protein comprising one protein to be produced in the form of PCIs fused to the selected PCI-forming protein.

In preferred embodiments, the nucleic acid molecule being selected from the group consisting of the nucleic acid molecules as referred to herein in items (i) to (xii) is located 5' of the further nucleic acid molecule, said further nucleic acid molecule encoding at least one protein (and in case of option (I) one) to be produced in the form of PCIs. In other preferred embodiments, the fusion construct comprises the endogenous promoter of the nucleic acid molecule being selected from the group consisting of the nucleic acid molecules as referred to herein in items (i) to (iii). The endogenous promoter in these preferred embodiments guides transcription of the nucleic acid molecules. These preferred embodiments with respect to the 5' to 3' orientation of the nucleic acid molecules within the fusion construct and the selection of the promoter do not only apply to fusion constructs according to option (I) but also apply to the below described options (II) and (III) of the fusion constructs.
Pursuant to option (II) the fusion construct may also comprise one nucleic acid molecule encoding two or more proteins to be produced in the form of PCIs being fused to a nucleic acid molecule being selected from the group consisting of the nucleic acid molecules as referred to herein in items (i) to (xii). In this case the fusion construct encodes a fusion protein, wherein the two or more proteins to be produced in the form of PCIs are fused to each other and are furthermore all fused to the protein encoded by the selected nucleic acid molecule as referred to herein in items (i) to (xii). Such a fusion construct may be, for example, a pBAD vector comprising an expression cassette encoding a CipA-GFP-LacZ fusion protein, a CipB-GFP-LacZ fusion protein or a PixA-GFP-LacZ fusion protein. Thus, it is be understood that the fusion construct pursuant to option (II) encodes at least one fusion protein, said fusion protein comprising at least two proteins to be produced in the form of PCIs being fused to each other and further being fused to the selected PCI-forming protein.

Moreover, in accordance with option (III) the fusion construct may comprise two or more nucleic acid molecules, wherein each of said nucleic acid molecules encodes at least one protein and preferably only one protein to be produced in the form of PCIs being fused to a nucleic acid molecule being selected from the group consisting of the nucleic acid molecules as referred to herein in items (i) to (xii). In this case the fusion construct encodes two or more fusion proteins, wherein each of the fusion proteins is a fusion protein wherein at least one protein and preferably only one protein to be produced in the form of PCIs is fused to the protein encoded by the selected nucleic acid molecules as referred to herein in items (i) to (xii). Hence, under option (III) the fusion construct harbors at least two expression cassettes, each encoding a fusion protein. For instance, a first expression cassette may encode a CipA-GFP-LacZ fusion protein, a CipB-GFP-LacZ fusion protein or a PixA-GFP-LacZ and a second expression cassette may encode a CipA-RFP-LacZ fusion protein, a CipB-RFP-LacZ fusion protein or a PixA-RFP-LacZ, noting that RFP is red fluorescent protein. Such a fusion construct is furthermore illustrated by the vector pBAD with a first expression cassette encoding CipA-GFP and a second expression cassette encoding CipA-LacZ, or the vector pBAD with a first expression cassette encoding CipB-GFP and a second expression cassette encoding CipA-LacZ. Pursuant to option (III) it is to be understood that the fusion construct encodes at least two distinct fusion proteins, wherein each of said fusion proteins comprises at least one protein to be produced in the form of PCIs fused to a selected PCI-forming protein.

Under option (I) the requirement of producing at least two proteins in the form of PCIs in a bacterial host cell is fulfilled in case two or more fusion constructs are introduced into the bacterial host cell. This is because under option (I) each fusion construct encodes only one protein to be produced, so that two fusion constructs are required for producing two proteins in the bacterial host cell. On the other hand, under options (II) and (III) the requirement of producing at least two proteins in the form of PCIs in a bacterial host cell is fulfilled by introducing only one such fusion construct into the bacterial host cell. This is because under options (II) and (III) each fusion construct already encode two distinct proteins to be produced, so that one fusion construct is sufficient for producing two proteins in the bacterial host cell. For this reason the use of fusion constructs pursuant to options (II) and (III) is preferred in case at least two proteins are to be produced in the form of protein crystalline inclusions (PCls) in a bacterial host. In addition, among options (II) and (III) fusion constructs pursuant to option (III) are preferred, as under option (III) each protein to be produced in the form of protein crystalline inclusions (PCIs) can be separately fused to a PCI-forming protein. Option (III) makes the system more flexible. For example, for each protein to be produced from a fusion construct according to option (III) a desired PCI-forming protein or a promotor to drive expression can be selected separately. The at least two proteins to be produced in the form of PCIs are with increasing preference at least three proteins, at least four proteins, and at least five proteins. It is to be understood that in case at least three or four proteins are produced in the form of PCIs more than one of the above three options may be used. For example, in case of three proteins one protein may be produced from a fusion construct under option (I) and the two other proteins from a fusion construct under option (II) or (III). In the case of four proteins, for example, one protein may be produced from a fusion construct under option (I) and the three other from a fusion construct under option (II) or (III), or two proteins may be produced from two fusion constructs under option (I) and the other two proteins from a fusion construct under option (II) or (III). In the case of four proteins also two proteins may be produced from a fusion construct under option (III) and the two other from a further fusion construct also falling under option (III).

As regards the required selection of a nucleic acid molecule being from the group consisting of the nucleic acid molecules as referred to herein in items (i) to (xii) it is preferred that all nucleic acid molecules are selected from items (i), (iv), (vii) and (x) (i.e. nucleic acid molecules encoding CipA or derivatives thereof), or items (ii), (v), (viii) and (xi) (i.e. nucleic acid molecules encoding CipB or derivatives thereof), or items (iii), (vi), (ix) and (xii) (i.e. nucleic acid molecules encoding PixA or derivatives thereof). According to these preferred embodiments the nucleic acid molecules encoding the PCI-forming proteins are selected such that all of the at least two proteins to be produced are fused to CipA or a derivative thereof, or to CipB or a derivative thereof, or to PixA or a derivative thereof. As discussed herein, CipA, CipB and PixA are independently and alone capable of forming PCls. However, using only one of CipA, CipB and PixA (or derivatives thereof) in the context of the present invention might be advantageous. For example, the conditions for the cell culture or expression might be selected such that they are optimized for the particular and selected PCI-forming protein. Among items (i), (iv), (vii) and (x) items (i) and (vii) are preferred (i.e. the nucleic acid molecule encoding CipA), among (ii), (v), (viii) and (xi) items (ii) and (viii) are preferred (i.e. the nucleic acid molecule encoding CipB), and among items (iii), (vi), (ix) and (xii) items (iii) and (ix) are preferred (i.e. the nucleic acid molecule encoding PixA). These more preferred options exclude derivatives thereof of the naturally occurring *cipA, cipB* and *pixA* nucleic acid molecules, respectively.

The fusion construct of the invention is preferably comprised in a vector. Preferably, the vector is a plasmid, cosmid, virus, bacteriophage or another vector used conventionally e.g. in genetic engineering. The fusion construct of the present invention may be inserted into several commercially available vectors. The fusion construct of the present invention referred to above may also be inserted into vectors such that a translational fusion with another nucleic acid molecule is generated. The other nucleic acid molecules may e.g. encode a protein that facilitates the purification of the protein encoded by the nucleic acid molecule of the invention or a protein of interest that is to be observed by fluorescence imaging. The vectors may also contain an additional expressible polynucleotide coding for one or more proteins to facilitate correct protein folding.

For vector modification techniques, see Sambrook and Russel, 2001, Molecular Cloning, 4th ed. CSH. Generally, vectors can contain one or more origins of replication (ori) and inheritance systems for cloning or expression, one or more markers for selection in the host, e.g., antibiotic resistance, and one or more expression cassettes. The coding sequences inserted in the vector can e.g. be synthesized by standard methods, or isolated from natural sources. Ligation of the coding sequences to transcriptional regulatory elements and/or to other amino acid encoding sequences can be carried out using established methods. Transcriptional regulatory elements (parts of an expression cassette) ensuring expression in bacterial host cells are well-known to those skilled in the art. These elements comprise regulatory sequences ensuring the initiation of transcription (e.g., translation initiation codon, promoters, such as naturally-associated or heterologous promoters and/or insulators). Additional regulatory elements may include transcriptional as well as translational enhancers. Preferably, the fusion construct of the invention is operatively linked to such expression control sequences allowing expression in a bacteria host cells. The vector may further comprise a selectable marker. Examples of selectable markers include neomycin, ampicillin, chloramphenicol, hygromycine, gentamicin, kanamycin, rifampicin resistance and the like.

Suitable bacterial expression vectors that can be used in connection with the present invention are known in the art. Non-limiting examples of bacterial expression vectors that can be used herein are pACYC177, pASK75, pBAD/His A, pBAD/His B, pBAD/His C, pBAD/MCS, pBADM-11, pBADM-20, pBADM-20(+), pBADM-30, pBADM-30(+), pBADM-41(+), pBADM-52, pBADM-52(+), pBADM-60, pBADM-60(+), pBAT4, pBAT5, pCal-n, pET-3a, pET-3b, pET-3c, pET-3d, pET-12a, pET-14b, pET-15b, pET-16b, pET-19b, pET-20b(+), pET-21d(+), pET-22b(+), pET-24d(+), pET-28a, pET-28c, pET-32a(+), pET-32b(+), pET-32c(+), pET-39b(+), pET-40b(+), pETM-10, pETM-11, pETM11-SUMO3GFP, pETM-12, pETM-13, pETM-14, pETM-14_ccdB, pETM-20, pETM-21, pETM-22, pETM-22_ccdB, pETM-30, pETM-33, pETM-33_ccdB, pETM-40, pETM-41, pETM-43, pETM-44, pETM-44_ccdB, pETM-50, pETM-51, pETM-52, pETM-54, pETM-55, pETM-60, pETM-66, pETM-70, pETM-80, pETM-82, pGAT, pGAT2, pGEX-3X, pGEX-4T-1, pGEX-4T-2, pGEX-4T-3, pGEX-5X-1, pGEX-5X-2, pGEX-5X-3, pGEX-6P-1, pGEX-6P-2, pGEX-6P-3, pHAT, pHAT2, pKK223-3, pKK223-2, pMal-c2, pMal-p2, pProEx HTa, pProEx HTb, pProEx HTc, pQE-16, pQE-30, pQE-31, pQE-32, pQE-60, pQE-70, pQE-80L, pQE-81L, pQE-82L, pRSET A, pRSET B, pRSET C, pTrcHis2 A, pTrcHis2 B, pTrcHis2 C, pTrcHis2LacZ, pZA31-Luc, pZE12-Luc, pZE21-MCS-1, and pZS*24-MCS-1. Within this list of vectors pBAD vectors are preferred as they were used in the examples herein below. As suitable hosts cells inter alia *Bacillus brevis, Bacillus megaterium, Bacillus subtilis, Caulobacter crescentus,* other strains, and, most importantly, *Escherichia coli* BL21 and *Escherichia coli* K12 and their derivatives can be named. As promoters are, for example, the L-arabinose inducible *araBAD* promoter (P_{BAD}), the *lac* promoter, the L-rhamnose inducible *rhaP_{BAD}* promoter, the T7 RNA polymerase promoter, the *trc* and tac promoter, the lambda phage promoter *p_{L},* and the anhydrotetracycline-inducible *tetA* promoter/operator.

The co-transformation with a selectable marker such as kanamycin or ampicillin resistance genes for culturing in *Escherichia coli* and other bacteria allows the identification and isolation of the transformed cells.

Selectable marker genes for mammalian cell culture include the dhfr, gpt, neomycin, hygromycin resistance genes. The transfected nucleic acid can also be amplified to express large amounts of the encoded (poly)peptide. The DHFR (dihydrofolate reductase) marker is useful to develop cell lines that carry several hundred or even several thousand copies of the gene of interest. Another useful selection marker is the enzyme glutamine synthase (GS) (Murphy et al. 1991; Bebbington et al. 1992). Using such markers, the cells are grown in selective medium and the cells with the highest resistance are selected.

Means and methods for introducing a fusion construct into a bacterial host cell are well known to the person skilled in the art. Non-limiting examples are transduction, transformation, conjugation or transfection. Transformation is the process by which a donor DNA molecule is taken up from the external environment and incorporated into the genome of the recipient cell. Transduction involves transfer of genetic material from one bacterium to another by a bacteriophage. Acting as a vector, the phage carries its own genome plus a fragment of DNA from the bacterium it has recently infected. If the host bacterium survives the viral attack, recombination may occur. Conjugation is the temporary direct contact between two bacterial cells leading to an exchange of DNA. This exchange is unidirectional, i.e. one bacterial cell is the donor of DNA and the other is the recipient. In this way, genes are transferred laterally amongst existing bacterial as opposed to vertical gene transfer in which genes are passed on to offspring. Transfection is the process of deliberately introducing naked or purified nucleic acids by non-viral methods into cells. Transfection can, inter alia, be carried out using calcium phosphate (i.e. tricalcium phosphate), by electroporation, by cell squeezing or by mixing a cationic lipid with the material to produce liposomes which fuse with the cell membrane and deposit their cargo inside.

Suitable conditions for culturing a bacterial host cell are well known to the person skilled in the art. For example, suitable conditions for culturing bacteria include growth under aeration in Lysogenic Broth (LB) medium. To increase the yield and the solubility of the expression product, the medium can be buffered or supplemented with suitable additives known to enhance or facilitate growth. For example, *Escherichia coli* can be cultured from 4 to about 37 °C, the exact temperature or sequence of temperatures depending on the molecule to be overexpressed. The skilled person is also aware of all these conditions and may further adapt these conditions to the needs of a particular host species and the requirements of the proteins to be produced. In case an inducible promoter controls the nucleic acid to be expressed via the vector present in the host cell, synthesis of the protein can be induced by addition of an appropriate inducing agent.
Provided herein are novel scaffolds that can organize proteins in PCIs and methods using the same. As is demonstrated in the examples herein below, it was surprisingly and advantageously found that the desired proteins in the form of PCIs (i) retain or even improve the original protein activities in the cell despite being organized in PCls, (ii) remain stable during product synthesis, and (iii) can easily be isolated for further applications. In more detail, it is demonstrated in the examples herein below that CipA and CipB can be used as scaffolds to organize other desired proteins into subcellular structures, so-called PCls. It was also surprisingly found in connection with the present invention that Cip scaffolds can organize multiple proteins into PCIs in the native host *Photorhabdus luminescens,* but also do so heterologously in *Escherichia coli.* PCIs based on Cip scaffolds have diverse applications. On the one hand, they can be readily isolated and utilized for further applications because they are insoluble in water and resistant to mechanical cell disruption. On the other hand and as also illustrated in the examples herein below, Cip scaffolds can organize multiple enzymes into functional complexes *in vivo* and are therefore also useful for metabolic engineering.

In accordance with a preferred embodiment of the first aspect of the invention, the method further comprises purifying the PCIs from the bacterial host cell.

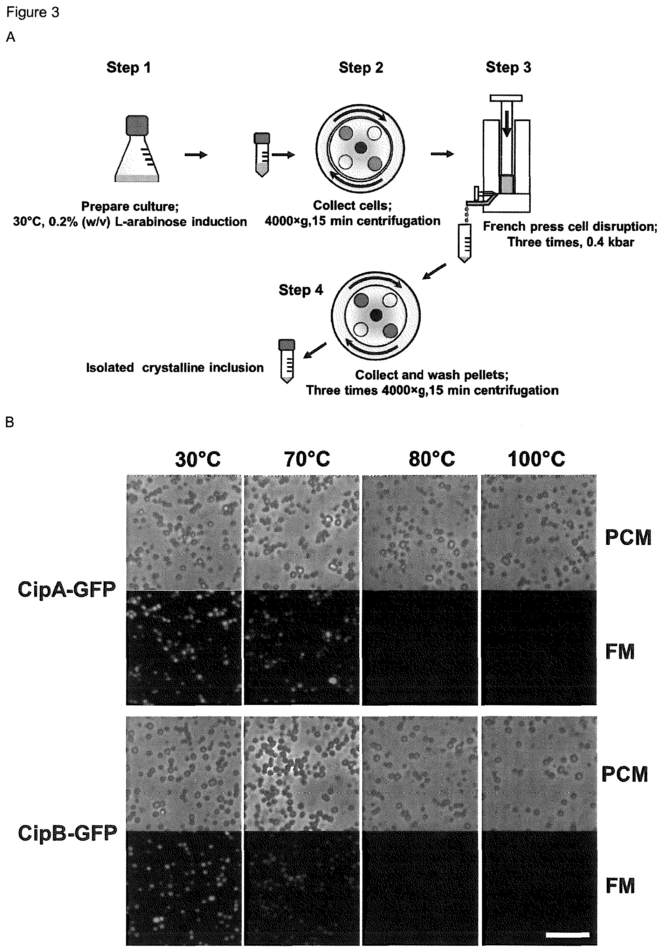

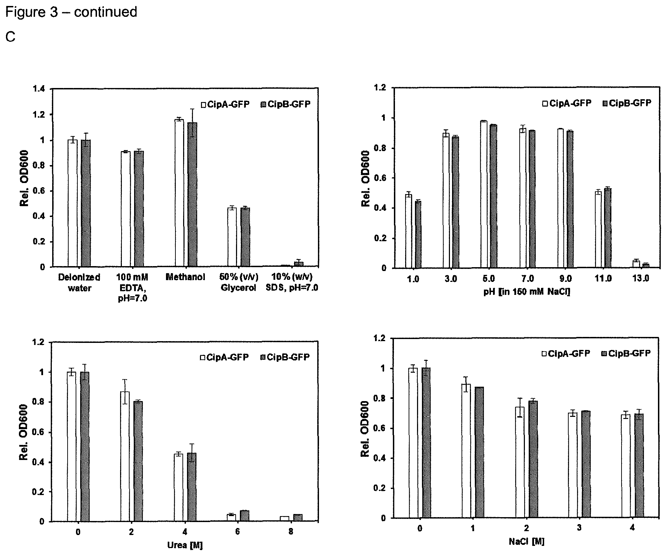

Protein purification, in this embodiment of the PCls, in accordance with the invention specifies a process or a series of processes intended to further isolate the polypeptide of the invention from a complex mixture preferably to homogeneity. Purification steps, for example, exploit differences in protein size, physico-chemical properties and binding affinity. Methods of purification of the proteins produced are well-known in the art and comprise without limitation method steps such as selective centrifugation, ion exchange chromatography, gel filtration chromatography (size exclusion chromatography), affinity chromatography, high pressure liquid chromatography (HPLC), reversed phase HPLC, disc gel electrophoresis or immunoprecipitation. Such methods are also illustrated in the examples herein below, in particular in Example 3 and Figure 3.

Proteins may be purified according to their isoelectric points by running them through a pH graded gel or an ion exchange column. Further, proteins in this embodiment of the PCIs may be separated according to their size or molecular weight via size exclusion chromatography or by SDS-PAGE (sodium dodecyl sulfate-polyacrylamide gel electrophoresis) analysis. In the art, proteins are often purified by using 2D-PAGE and are then further analysed by peptide mass fingerprinting to establish the protein identity. This is very useful because the detection limits for proteins are very low and nanogram amounts of protein are in general sufficient for their analysis. Proteins in this embodiment of the PCIs may also be separated by polarity/hydrophobicity via high performance liquid chromatography or reversed-phase chromatography. Most preferably, the PCIs are purified to more than 98%, such as 99% or 100% purity, i.e. they are free or essentially free of contaminants.

In accordance with a further preferred embodiment of the first aspect of the invention, the method further comprises purifying the proteins from the PCIs.

Also means and methods for purifying the produced proteins from the PCIs are well-known to the skilled person. For example, an enzyme recognition site may be used to fuse the protein(s) to be produced in the form of PCIs and the PCI-forming protein which enables the separation of the protein(s) to be produced in the form of PCIs from the PCI-forming protein by enzymatic digestion.

Methods for protein purification have been described herein above and may equally be used in connection with this further preferred embodiment.

In accordance with a still further preferred embodiment of the first aspect of the invention, the least two proteins are at least two proteins involved in the same multi-step biosynthetic pathway.

Biosynthesis is usually a multi-step, enzyme-catalyzed process where substrates are converted into more complex products in living organisms, including prokaryotic and eukaryotic cells. Along a multi-step biosynthetic pathway, simple compounds are modified, converted into other compounds, or joined together to form the desired compound, often being a protein. Some of these biosynthetic pathways are located within a single cellular organelle, while others involve enzymes that are located within multiple cellular organelles. Examples of these biosynthetic pathways include the production of lipid membrane components and nucleotides.

The required elements for biosynthesis include: precursor compounds, chemical energy (e.g. ATP), and catalytic enzymes which may require coenzymes (e.g. NADH, NADPH). These elements usually create monomers, the building blocks for the desired compound. In other terms, precursor compounds are the starting substrates in a reaction. These may also be viewed as the reactants in a given chemical process. Chemical energy can be found in the form of high energy molecules. These molecules are required for energetically unfavorable reactions. Furthermore, the hydrolysis of these compounds drives a reaction forward. High energy molecules, such as ATP, usually have three phosphates. Often, the terminal phosphate is split off during hydrolysis and transferred to another molecule. Catalytic enzymes are special proteins that catalyze a reaction by increasing the rate of the reaction and lowering the activation energy. Coenzymes or cofactors are molecules that assist in chemical reactions. These may, inter alia, be metal ions, vitamin derivatives such as NADH and acetyl CoA, or non-vitamin derivatives such as ATP. In the case of NADH, the molecule transfers a hydrogen, whereas acetyl CoA transfers an acetyl group, and ATP transfers a phosphate.

Hence, it is to be understood in connection with this embodiment that the at least two proteins to be produced in the form of PCIs are preferably the catalytic enzymes that catalyze a reaction by increasing the rate of the reaction and lowering the activation energy. However, the proteins may also be coenzymes or cofactors. It is preferred that the at least proteins are all proteins being required in order to produce the one or more desired compound.

In a further preferred embodiment of the first aspect of the invention, the present invention relates to a method further comprising producing at least one compound being a product of said multi-step biosynthetic pathway wherein all proteins being required for the production of said at least one compound are contained in said bacterial host cell in expressible form and optionally further comprising purifying the at least one compound from the bacterial host cell.

It is to be understood that the bacterial host cell comprises or has access to all required substrates for synthesizing the at least one compound being produced via the multi-step biosynthetic pathway.

The invention thus also relates to a method for the production of at least one compound being produced via a multi-step biosynthetic pathway, wherein the method comprises producing all of the proteins being required in the multi-step biosynthetic pathway for the production of the at least one compound by (a) producing at least two and preferably all of the proteins in the form of PCIs by introducing into the bacterial host cell one or more fusion constructs, said one or more fusion constructs comprising in expressible form one or more nucleic acid molecules encoding the at least two and preferably all of said proteins, wherein each of the one or more nucleic acid molecules is independently fused to a nucleic acid molecule being selected from the group of nucleic acid molecules pursuant to items i. to xii. as defined herein above; and (b) provided that the fusion construct as defined in step (a) does not encode all of said proteins, producing all other of said proteins in a PCI-free form by introducing into the bacterial host cell at least one nucleic acid molecule encoding in producible form all other of said proteins; and (c) culturing the bacterial host cell obtained after step (b) to synthezise the at least one compound, wherein the bacterial host cell comprises or has access to the substrate(s) of the at least one compound being produced via the multi-step biosynthetic pathway. This embodiment also forms part of the invention.

The preferred embodiments, definitions and explanations described herein above in connection with the first aspect of the invention as far as being applicable to the above embodiment of the invention apply *mutatis mutandis.* For instance, also in connection with the step of producing at least two and preferably all of the proteins in the form of PCIs by introducing into the bacterial host cell one or more fusion constructs, fusion constructs according to the above discussed options (I), (III) and (III) may be used and the preferred options as regards the selection from items (i) to (xii) equally apply. It is to be understood that the proteins to be produced in accordance with the first aspect of the invention are in accordance with the preferred embodiment of the invention all of the proteins being required in the multi-step biosynthetic pathway for the production of the at least one compound.

As discussed, the compound being produced via the multi-step biosynthetic pathway is not particularly limited and may be a protein or nucleotide, preferably a protein. The proteins being required in the multi-step biosynthetic pathway for the production of the at least one compound may be enzymes that catalyze the reactions being involved in the multi-step biosynthetic pathway leading to the desired product to be produced and/or coenzymes or cofactors required in this multi-step biosynthetic pathway. The substrate(s) are the starting compounds being processed into the desired compound along the multi-step biosynthetic pathway, usually via one or more intermediate compounds.

In accordance with the method according to this preferred embodiment it may be required to produce some of the proteins being required or desired in the multi-step biosynthetic pathway for the synthesis of the at least one compound in a PCI-free form. In this respect the term "PCI-free" means that upon the production of the respective protein in the bacterial host cell the protein cannot be found in the protein crystalline inclusions. Hence, those nucleic acid molecule encoding in expressible form these proteins are not fused to the nucleic acid molecules according to any one of items (i) to (xii). Rather, these are typically expressed from conventional vectors as described above. In another preferred embodiment, all proteins involved in the multistep biosynthetic pathway are produced in the form of PCIs according to the invention.

More preferably, the at least one compound is either an intermediate or an end-product of the multi-step biosynthetic pathway.
If the compound is an intermediate, then only those proteins need to be produced in the bacterial host cell that are required to synthesize the compound. In accordance with the invention, at least two of those proteins need to be produced in the form of PCIs. On the other hand, if the end-product of such a pathway is to be synthesized, then all proteins involved in this pathway need to be produced, at least two of them in the form of PCIs.

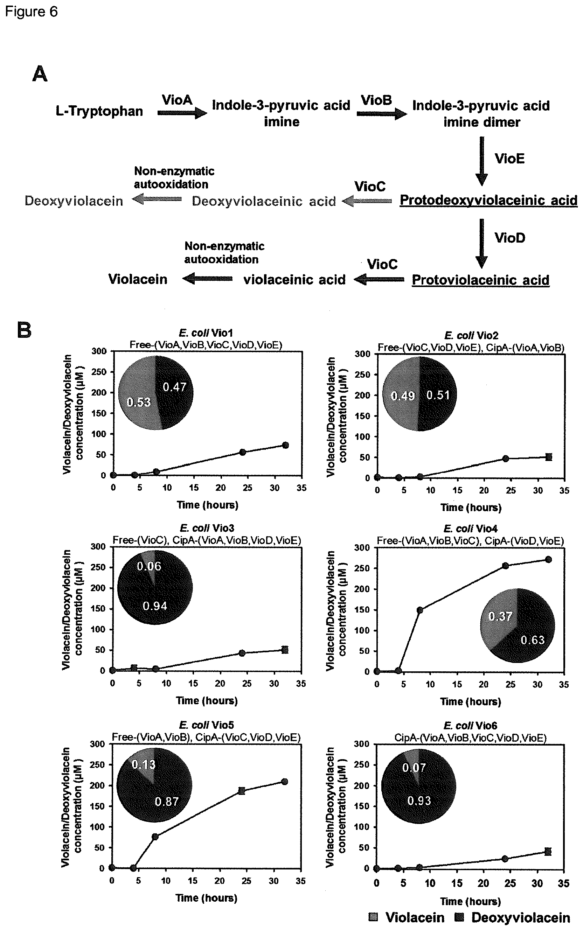

As is shown in the examples of the specification, on the basis of the five Vio enzymes (i.e. VioA, VioB, VioC, VioD and VioE) being required for the production of the desired compound violacein it has been surprisingly found that producing, in accordance with the invention, at least two and preferably all of the proteins being required for synthesis of a desired compound via a multi-step biosynthetic pathway in bacteria host cells advantageously allows for controlling and orchestrating the biosynthesis in advantageous manner. In more detail, violacein is produced through a 5-step enzymatic process from L-tryptophan via the intermediate products indole-3-pyruvic acid imine, protodeoxyviolaceinic acid, protoviolaceinic acid and violaceinic acid. In an undesired side reaction deoxyviolacein may be produced from the intermediate product protodeoxyviolaceinic acid. As shown in the first graph of Figure 6B when producing all five Vio enzymes in a PCI-free form relatively low amounts of violacein/deoxyviolacein are obtained and in addition 53% of the synthesized violacein/deoxyviolacein are the undesired side product deoxyviolacein. On the other hand and as also shown in Figure 6B when producing four or all five or the Vio enzymes in the form of PCIs more than 90% of the produced violacein/deoxyviolacein is the desired violacein. Furthermore, Figure 6B shows that when producing two or three of the Vio enzymes in the form of PCIs the synthesized amount of violacein/deoxyviolacein significantly increases and in addition to that also a higher percentage of violacein is obtained.
In accordance with a further preferred embodiment of the first aspect of the invention, the method further comprises purifying the at least one compound from the bacterial host cell.

Means and methods for purifying a compound from a bacterial host cell are discussed herein above in connection with the main embodiment of the invention. These methods can also be used in connection with this preferred embodiment of the invention.

In accordance with a further preferred embodiment of the first aspect of the invention, the proteins are enzymes.

As also mentioned, the proteins being required in the multi-step biosynthetic pathway for the production of the at least one compound may be enzymes that catalyze the reactions being involved in the multi-step biosynthetic pathway leading to the desired product to be produced and/or coenzymes or cofactors required in this multi-step biosynthetic pathway. As many multi-step biosynthetic pathways do not require coenzymes or cofactors or only comprise coenzymes or cofactors being naturally produced and thus present in the bacterial cost cell, the proteins being required in the multi-step biosynthetic pathway for the production of the at least one compound are preferably enzymes.

In accordance with a more preferred embodiment of the invention, the enzymes are involved in a biosynthetic pathway to produce a primary or secondary metabolite and/or to increase the yield of such a metabolite.

The advantageous effects obtained by controlling and orchestrating the biosynthesis of violacein via PCIs are not limited to violacein biosynthesis but can be transferred to and obtained for further compounds being produced via a multi-step biosynthetic pathway. Many multi-step biosynthetic pathways result in the production of primary or secondary metabolites or at least increase the yield of such a metabolite (e.g. by positive feed-back loops). A metabolite is a product of metabolism, wherein metabolism refers to the chemical changes in a living cell by which energy is provided for vital processes and activities and new material is assimilated. A primary metabolite is a kind of metabolite that is directly involved in normal growth, development, and reproduction. It usually performs a physiological function in the organism (i.e. an intrinsic function). A secondary metabolite is an organic compound that is not directly involved in the normal growth, development, or reproduction of an organism. Unlike primary metabolites, absence of secondary metabolites does not result in immediate death, but rather in long-term impairment of the organism's survivability, fecundity, or aesthetics, or perhaps in no significant change at all. Non-limiting examples of primary metabolites are amino acids, sugars, alcohols, lipids, aromatic compounds, steroids and co-enzymes. Non-limiting examples of secondary metabolites are antibiotics, cancerostatics, fungicides, insecticides, pesticides, dyes and hormones. The metabolites are usually end-products of the multi-step biosynthetic pathways.

In accordance with a further more preferred embodiment of the invention, the enzymes are selected from VioA, VioB, VioC, VioD and VioE.

As discussed herein above, the five Vio enzymes are used in the examples herein below in order to illustrate the invention. For this reason the enzymes being required in the multi-step biosynthetic pathway for the production of the at least one compound are preferably the five Vio enzymes.

In accordance with an even more preferred embodiment of the first aspect of the invention, (i) VioD and VioE are produced in the form of PCls, and VioA, VioB and VioC are produced in PCI-free form, (ii) VioC, VioD and VioE are produced in the form of PCls, and VioA and VioB are produced in PCI-free form, or (iii) three, and preferably four or all five of VioA, VioB, VioC, VioD and VioE are produced in the form of PCls.

Hence with respect to the above preferred embodiment of the first aspect of the invention that was formulated herein as independent embodiment in accordance with an even more preferred embodiment of the invention (i) the fusion construct of step (a) comprises a nucleotide sequence encoding VioD and VioE, and the at least one nucleotide sequence of step (b) encodes VioA, VioB and VioC, (ii) the fusion construct of step (a) comprises a nucleotide sequence encoding VioC, VioD and VioE, and the at least one nucleotide sequence of step (b) encodes VioA and VioB, or (iii) the fusion construct of step (a) comprises a nucleotide sequence encoding three, and preferably four or all five of VioA, VioB, VioC, VioD and VioE.

As shown by Figure 6B, producing the five Vio enzymes as required by items (i) and (ii) significantly increases the synthesized amount of violacein/deoxyviolacein and in addition increases the percentage of violacein comprised in the produced amount. As also depicted in Figure 6B, producing the five Vio enzymes as required by item (iii) significantly increases the percentage of violacein comprised in the produced amount.

In accordance with another even more preferred embodiment of the second aspect of the invention, the at least one compound is violacein.

Violacein is known to have diverse biological activities, including anticancer activity and antibiotic activity against *Staphylococcus aureus* and other Gram-positive pathogens. The strong antibacterial effects make violacein a promising candidate as an antibiotic. The most studied clinical use of violacein, however, is it being a potential cancer therapeutic. Violacein has been tested against various cancer cell lines, where it has shown cytotoxicity at IC50 values that mostly range in the submicromolar concentrations. Violacein is naturally produced by diverse and phylogenetically distinct genera of bacterial strains, including *Chromobacterium, Collimonas, Duganella, Janthinobacterium.* Violacein is a secondary metabolite (see Choi et al. (2015), BioMed Research International, Article ID 465056, 8 pages).

In accordance with a preferred embodiment of the invention, the bacterial host is a proteobacterium, preferably a gammaproteobacterium, more preferably an enterobacterium, and is most preferably *Escherichia coli, Photorhabdus spec.,* or *Xenorhabdus spec.,* wherein the genes *cipA* and *cipB* of *Photorhabdus spec.,* and the gene *pixA* of *Xenorhabdus spec.* are inactivated.

In the examples herein below proteins were produced in the form of PCIs in *Escherichia coli* and *Photorhabdus spec.* host cells, wherein the genes *cipA* and *cipB* of *Photorhabdus spec.* were inactivated. Moreover, *Xenorhabdus spec.* is as *Photorhabdus spec.* naturally capable of producing PCIs. Although *Escherichia coli, Xenorhabdus spec.* and *Photorhabdus spec.* are evolutionary distinct bacterial species they are both proteobacteria, in particular gammaproteobacteria of the order enterobacteria. The host cell is most preferably an *Escherichia coli* cell.

In a second aspect the present invention relates to a fusion construct comprising in expressible form one or more nucleic acid molecules encoding at least two proteins to be produced in the form of PCIs, wherein each of the one or more nucleic acid molecules is independently fused to a nucleic acid molecule being selected from the group of nucleic acid molecules pursuant to items i. to xii. as defined herein above.

Hence, the preferred embodiments, definitions and explanations described herein above in connection with the first aspect of the invention as far as being applicable to the second aspect of the invention apply *mutatis mutandis.* For instance, also in connection with the second aspect of the invention the preferred options as regards the selection from items (i) to (xii) apply.

In a third aspect the present invention relates to a bacterial host cell comprising one or more fusion constructs as defined in connection with the method of the first aspect of the invention.

The above host cell is identical to the host cell that is used in the method of the first aspect of the invention. In accordance with a preferred embodiment of the third aspect of the invention the host cell is identical to the host cell that is used in the method of the first aspect of the invention.

The preferred embodiments, definitions and explanations described herein above in connection with the first aspect of the invention as far as being applicable to the third aspect of the invention apply *mutatis mutandis.* For instance, also in connection with the one or more fusion constructs of the host cell comprising in expressible form one or more nucleic acid molecules encoding at least two proteins to be produced in the form of PCls, the fusion constructs are in accordance with the above discussed options (I), (III) and (III) and the preferred options as regards the selection from items (i) to (xii) equally apply.

In a fourth aspect the present invention relates to a method for producing at least one protein in the form of PCIs in an *Escherichia coli* host cell, wherein the method comprises (a) introducing into the *Escherichia coli* host cell a fusion construct, said fusion construct comprising in expressible form a nucleic acid molecule encoding the least at least one protein fused to a nucleic acid molecule being selected from the group of nucleic acid molecules pursuant to items i. to xii. as defined herein above, and (b) culturing the *Escherichia coli* host cell obtained in (a) to produce the at least one heterologous protein in the form of PCIs.

The preferred embodiments, definitions and explanations described herein above in connection with the first aspect of the invention as far as being applicable to the fourth aspect of the invention apply *mutatis mutandis.* With respect to the fourth aspect of the invention it is to be noted, though, that also only one protein can be produced in the form of PCIs. In this case only one fusion construct according to the above discussed option (I) may be used. In case two or more proteins were to be produced in accordance with the fourth aspect of the invention fusion constructs according to the above discussed options (I), (III) and/or (III) may be used. Also, for example, the preferred options as regards the selection from items (i) to (xii) equally apply to the fourth aspect of the invention.

In the application CN101063101 one particular protein, namely bovine lactoferrin fused to CipB was produced in the form of PCIs in a host cell. As the host cell a mutant *Photorhabdus luminescens* cell was used, wherein the genes *cipA* and *cipB* of *Photorhabdus spec.* were deleted by homologous recombination. Hence, as the host cell a cell was used naturally forming PCls. The genes being responsible for the formation of PCIs were deleted and one of the genes has been reintroduced but being present the gene encoding bovine lactoferrin. In accordance with the fourth aspect of the present invention it was surprisingly found that PCIs are also formed in *Escherichia coli* host cells although these cells do not naturally produce PCIs. It was not foreseeable that the expression of only one gene knowing to form PCIs is sufficient in order to induce PCIs in a totally distinct bacterial host cell. In addition, *Escherichia coli* is the most commonly used and best studied bacterium which makes *Escherichia coli* cells most attractive as host cells.

As regards the embodiments characterized in this specification, in particular in the claims, it is intended that each embodiment mentioned in a dependent claim is combined with each embodiment of each claim (independent or dependent) said dependent claim depends from. For example, in case of an independent claim 1 reciting 3 alternatives A, B and C, a dependent claim 2 reciting 3 alternatives D, E and F and a claim 3 depending from claims 1 and 2 and reciting 3 alternatives G, H and I, it is to be understood that the specification unambiguously discloses embodiments corresponding to combinations A, D, G; A, D, H; A, D, I; A, E, G; A, E, H; A, E, I; A, F, G; A, F, H; A, F, I; B, D, G; B, D, H; B, D, I; B, E, G; B, E, H; B, E, I; B, F, G; B, F, H; B, F, I; C, D, G; C, D, H; C, D, I; C, E, G; C, E, H; C, E, I; C, F, G; C, F, H; C, F, I, unless specifically mentioned otherwise.

Similarly, and also in those cases where independent and/or dependent claims do not recite alternatives, it is understood that if dependent claims refer back to a plurality of preceding claims, any combination of subject-matter covered thereby is considered to be explicitly disclosed. For example, in case of an independent claim 1, a dependent claim 2 referring back to claim 1, and a dependent claim 3 referring back to both claims 2 and 1, it follows that the combination of the subject-matter of claims 3 and 1 is clearly and unambiguously disclosed as is the combination of the subject-matter of claims 3, 2 and 1. In case a further dependent claim 4 is present which refers to any one of claims 1 to 3, it follows that the combination of the subject-matter of claims 4 and 1, of claims 4, 2 and 1, of claims 4, 3 and 1, as well as of claims 4, 3, 2 and 1 is clearly and unambiguously disclosed.

The Figures show.

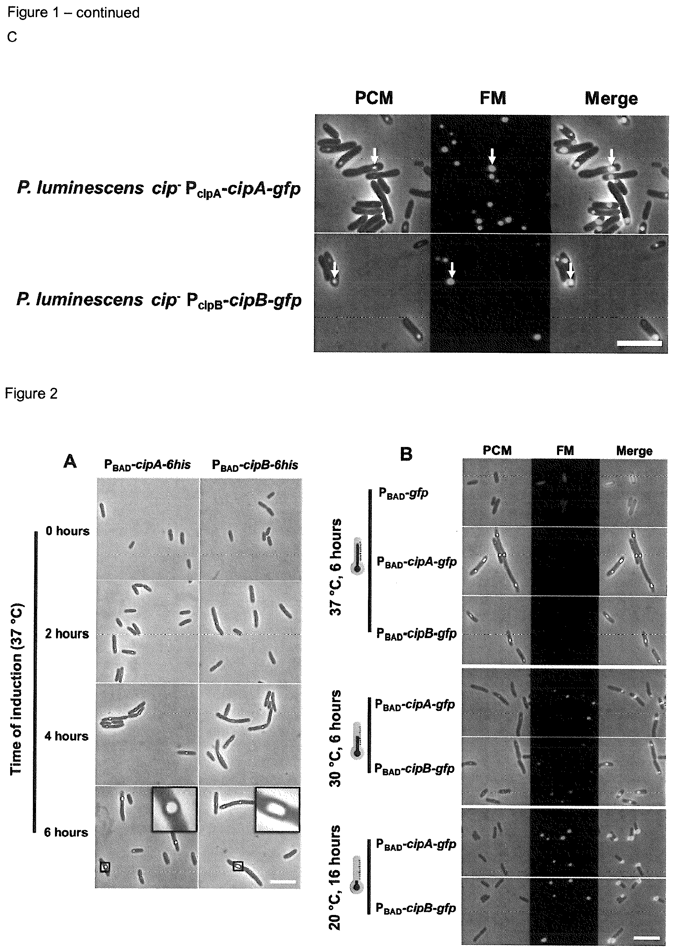

**Figure 1****. Cip scaffolds organize GFP into PCIs in *Photorhabdus luminescens.* (A)** Sequence comparison of CipA, CipB and PixA (amino acid identities are highlighted with boxes). **(B)** Relative abundance of individual amino acids in CipA, CipB and PixA. (hydrophobic, acidic and basic amino acids are underlined). **(C)** Both CipA-GFP and CipB-GFP hybrids are organized into PCIs in *Photorhabdus luminescens* TT01 *ΔcipA*/*ΔcipB* cells. The fusion genes *cipA-gfp* and *cipB-gfp* were expressed under the control of the native promoters of *cipA* and *cipB,* respectively. After 56 h of growth on LB plates at 30°C, *Photorhabdus luminescens* TT01 *ΔcipA*/*ΔcipB* cells producing CipA-GFP and CipB-GFP were collected and resuspended in fresh LB medium for microscopy. Arrows indicate PCIs. PCM, phase-contrast microscopy; FM, fluorescence microscopy. Scale bar, 7.5 µm.
**Figure 2****. Cip scaffolds organize GFP into subcellular structures in *Escherichia coli.* (A)** CipA-6His and CipB-6His self-organize into PCIs in *Escherichia coli* DH5α. Production of CipA-6His and CipB-6His in *Escherichia coli* DH5α was induced with 0.2% (w/v) L-arabinose at 37°C, when the cell density reached OD600=0.6. Cells were subsequently analyzed every 2 h by microscopy. The PCIs in the areas marked by the small black boxes (bottom row) are shown at higher magnification at the top right. **(B)** Production of GFP, CipA-GFP and CipB-GFP at 37°C was induced as in **(A).** In parallel, cells were cultivated at 30°C and 20°C. Micrographs were taken after induction for the indicated times (PCM, phase-contrast microscopy; FM, fluorescence microscopy). Scale bar, 7.5 µm.
**Figure 3****. Isolation and characterization of CipA-GFP and CipB-GFP PCIs. (A)** Schematic depiction of the procedure used for production and isolation of CipA-GFP and CipB-GFP PCIs. *Escherichia coli* cells were cultivated under inducing conditions at 30°C in LB medium. Cells were collected by centrifugation, and disrupted by three passages through a French press. The PCIs were then collected by centrifugation and washed three times. **(B)** Thermal stability of isolated PCIs. Micrographs were taken after a 10- min incubation at the indicated temperature (PCM, phase contrast microscopy; FM, fluorescence microscopy). Scale bar, 7.5 µm. **(C)** Solubility of PCI in various solutes. The degree of solubilization of PCIs was estimated by the relative decrease in initial optical density. The value for PCIs in deionized water was set to 1.0.

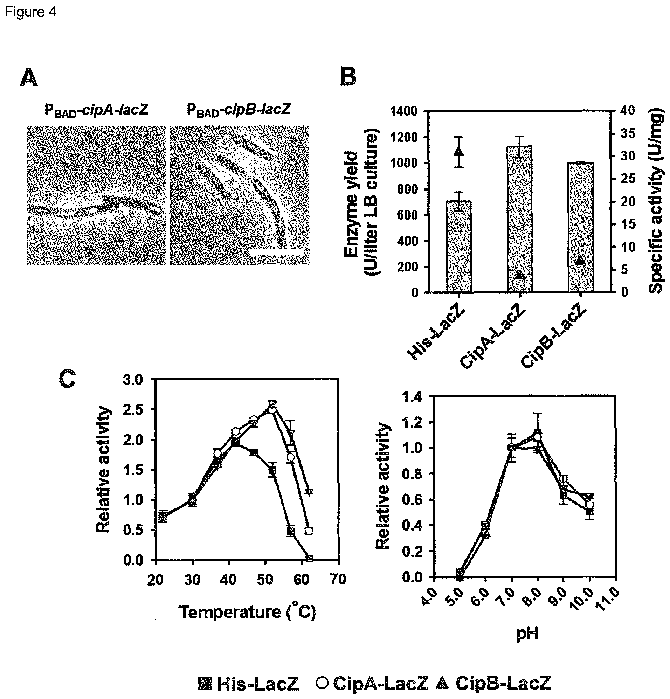

**Figure 4****. Cip scaffolds organize LacZ into PCIs in *Escherichia coli.* (A)** Micrographs of CipA-LacZ- or CipB-LacZ-producing *Escherichia coli* cells. Synthesis of CipA-LacZ or CipB-LacZ in *Escherichia coli* DH5α was induced with 0.2% (w/v) L-arabinose at 37°C for 6 h (scale bar, 7.5 µm). **(B)** Enzyme yield (grey column) and specific activity (black triangle) of His-LacZ, CipA-LacZ PCIs and CipB-LacZ PCIs. **(C)** Effects of temperature and pH on the activity of His-LacZ, CipA-LacZ PCIs and CipB-LacZ PCIs. The β-galactosidase activity measured at 30°C and pH 7.0, respectively, was set to 1.0.

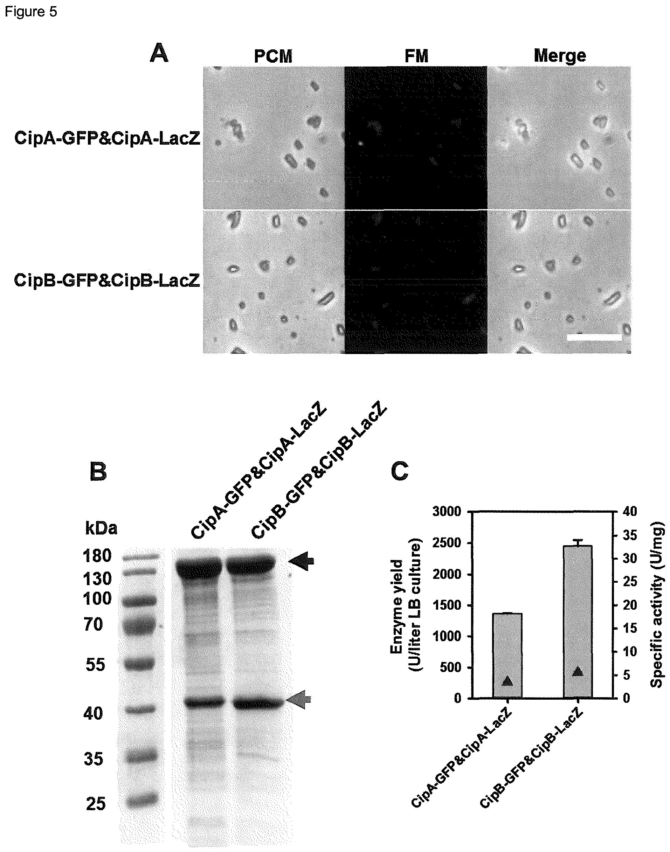

**Figure 5****. Cip scaffolds co-organize GFP and LacZ into PCIs in *Escherichia coli.* (A)** Isolated CipA-GFP&CipA-LacZ and CipB-GFP&CipB-LacZ PCIs. CipA-GFP and CipA-LacZ or CipB-GFP and CipB-LacZ were co-produced as PCls, which were released from the cells as described in Figure 2. Scale bar, 7.5 µm. **(B)** SDS-PAGE of CipA-GFP&CipA-LacZ and CipB-GFP&CipB-LacZ PCIs. The black arrow indicates CipA-LacZ or CipB-LacZ and the grey arrow indicates CipA-GFP or CipB-GFP. **(C)** β-galactosidase yields (grey column) of CipA-GFP&CipA-LacZ and CipB-GFP&CipB-LacZ PCIs and their corresponding specific activities (indicated by the black triangle).

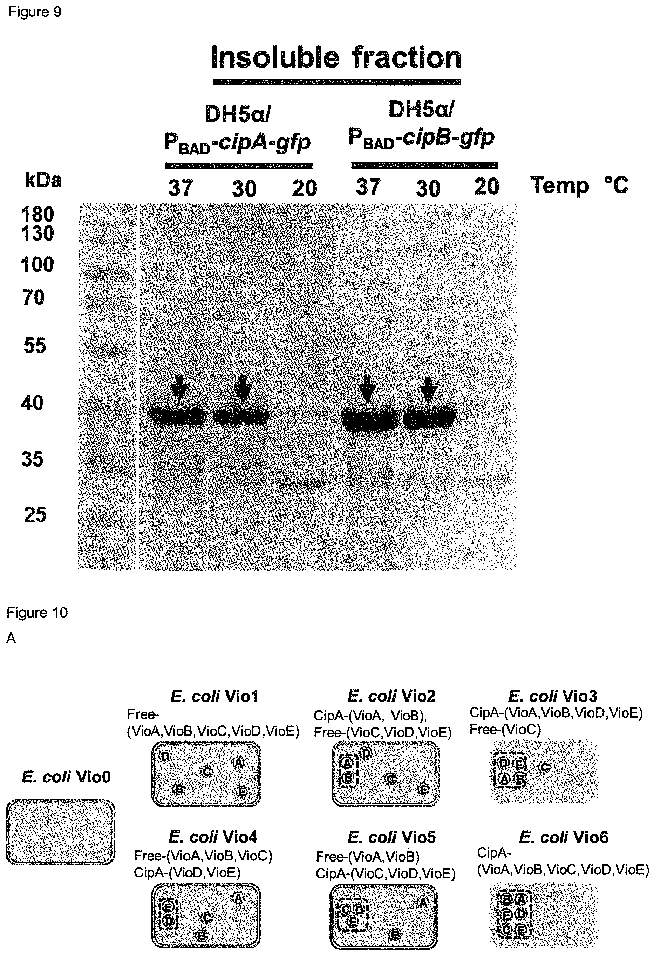

**Figure 6****. CipA can bring together multiple enzymes involved in violacein biosynthesis in *Escherichia coli.* (A)** The biosynthetic pathway that converts L-tryptophan into violacein. The underlined compounds are reported to be very unstable intermediates. (B) Time course of violacein and deoxyviolacein synthesis (mixture) in cultures of the engineered *Escherichia coli* strains. The pie charts indicate the ratios of violacein (black) to deoxyviolacein (grey) in the final product, as revealed by HPLC. Strain names and characteristics are given above the plots (see scheme in Figure 10A).

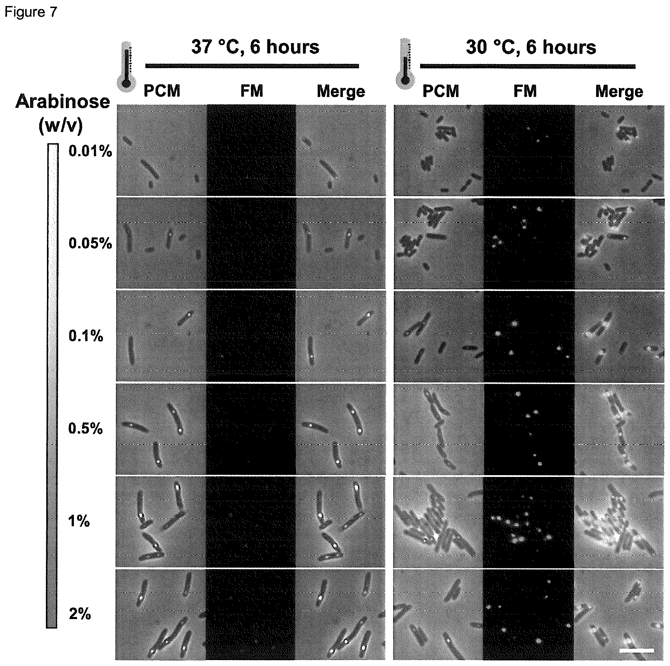

**Figure 7****. Influence of inducer concentration on formation of CipA-GFP PCIs in *Escherichia coli.*** The cultivation procedure (at 37°C or 30°C) was the same as described in Figure 2B, but different L-arabinose concentrations were tested; Bar, 7.5 µm.

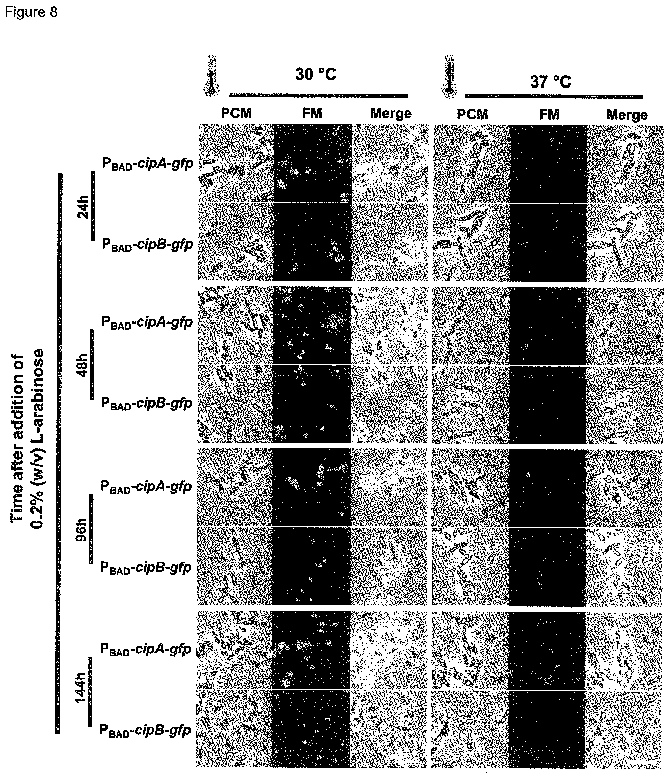

**Figure 8****. Stability of CipA-GFP and CipB-GFP PCIs in *Escherichia coli*** cells. Production of the CipA-GFP and CipB-GFP PCIs in *Escherichia coli was* initiated as described by addition of 0.2% (w/v) L-arabinose. Cultures were grown at 30°C and 37°C, respectively. Bar, 7.5 µm.
**Figure 9****. SDS-PAGE of the CipA-GFP and CipB-GFP protein complexes.** *Escherichia coli* cells were cultivated at 37°C, 30°C and 20°C to produce CipA-GFP and CipB-GFP as described in Figure 2. Cells were collected and lysed as described in Figure 3A. Insoluble fractions were collected and washed three times with Na-phosphate buffer, and resuspended in an equal final volume compared to the supernatant. Equal volumes (24 µL) of washed pellets and supernatants were loaded on the SDS-PAGE. Gels were stained with Coomassie Brilliant Blue R-250. Arrows marks CipA-GFP and CipB-GFP, respectively.

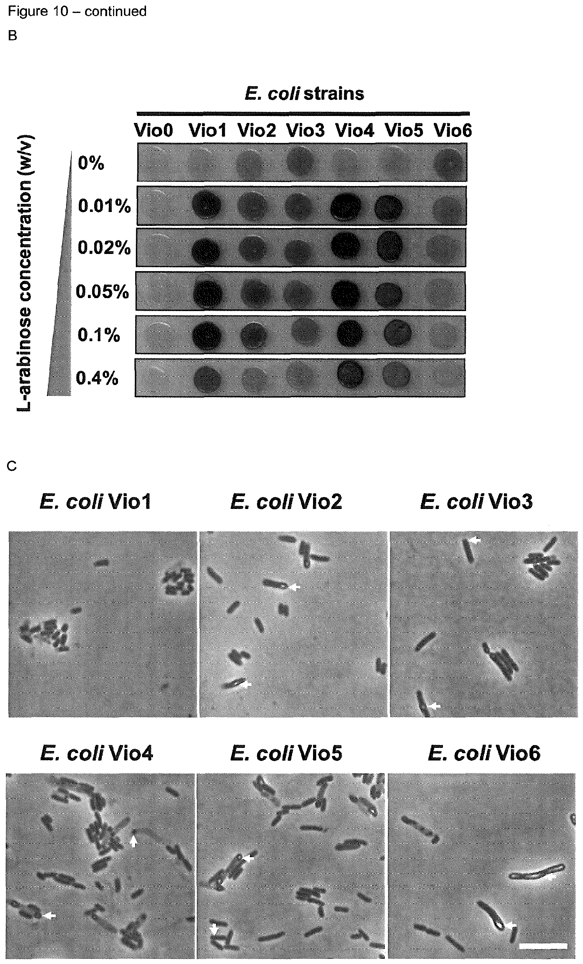

**Figure 10****. CipA scaffold organize violacein biosynthetic enzymes in *Escherichia coli.* (A)** Scheme of the constructed violaclein production strains: *Escherichia coli* Vio1 to *Escherichia coli* Vio6 as well as a control strain *Escherichia coli* Vio0 harboring empty vectors. **(B)** Selection of optimal L-arabinose inducer concentration. Precultures of the engineered strains in (A) were diluted with LB to an OD600 of 0.05 and dripped to LB plates containing indicated concentrations of L-arabinose and cultivated at 30°C overnight to stationary phase. The best inducer concentration was selected according to the pigment accumulation. **(C)** Vio enzymes were organized into PCIs (indicated by arrows) by CipA scaffold. Cells were collected at the 32h time point as depicted in Figure 6B and analyzed by microscope. Bar, 7.5 µm.

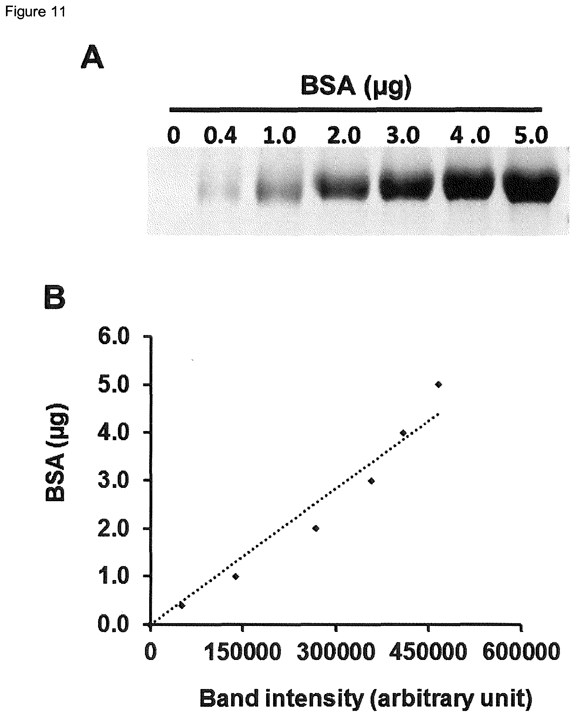

**Figure 11****. Calibration of ImageQuant TL with BSA standard. (A)** Different amounts of BSA (0, 0.4, 1, 2, 3, 4, 5 µg) were loaded on an SDS-PAGE gel. The SDS-gel was stained with Coomassie Brilliant Blue R-250 Dye. **(B)** Based on (A), a calibration curve between loaded BSA protein amount (µg) and band intensity (arbitrary units) (software **ImageQuant** TL) was used to calculate the protein amounts of the PCIs samples.

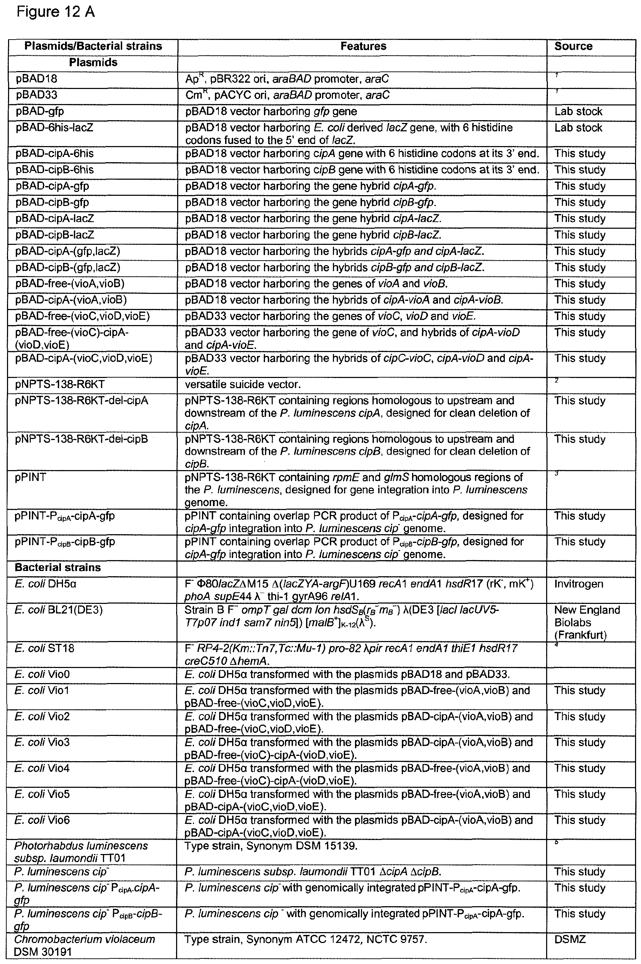

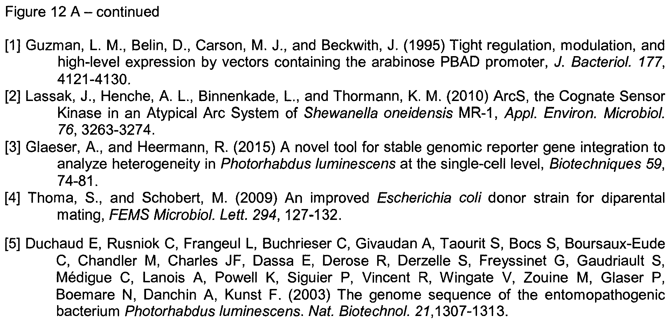

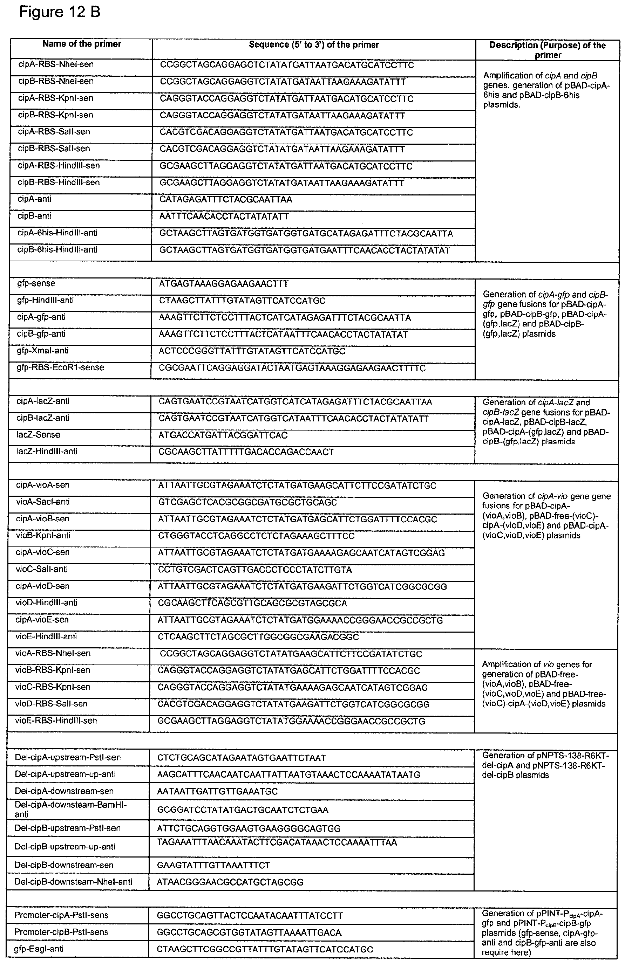

**Figure 12****. (A)** Overview of strains and plasmids used in the examples. **(B)** Primers used in the examples.

The Examples illustrate the invention.

### Example 1 - CipA and CipB as scaffolds in the native host Photorhabdus luminescens

CipA and CipB share 38% sequence similarity (Figure 1A), and form independent PCIs with different shapes in *Photorhabdus luminescens.^{19, 20}* The mechanism underlying their organization into PCIs is unknown. It is speculated herein that their hydrophobicity is the primary driving force, because both proteins contain high proportions of hydrophobic amino acids (CipA and CipB comprise, respectively, 49.0% and 51.0% hydrophobic amino acids; Figure 1B). First it was investigated whether CipA and CipB can function as protein scaffolds to organize exogenous proteins into PCIs in their native environment. As first step, a *cipA⁻ cipB⁻* double deletion mutant of *Photorhabdus luminescens* was generated, *Photorhabdus luminescens* TT01 *ΔcipA*/*ΔcipB (P. luminescens cip⁻*). The gene fusions *cipA-gfp* or *cipB-gfp* (under control of their respective native promoters) coding either for CipA-GFP or CipB-GFP was then integrated into the chromosome of this mutant, such that each was expressed from its native (*cip*) promoter. Then, the cells were grown for 56 h on LB agar at 30°C, and *Photorhabdus luminescens* cells producing fluorescent CipA-GFP or CipB-GFP PCls, respectively, were observed (Figure 1 C). This experiment proves that both CipA and CipB can act independently as scaffolds in their native host. In synchrotron X-ray tests of the resulting PCls, strong diffraction signals from CipB PCIs were obtained (produced by *Photorhabdus luminescens cipA⁻,* data not shown).

### Example 2 - CipA and CipB serve as scaffolds in Escherichia coli

Formation of CipA-GFP and CipB-GFP PCIs in *Photorhabdus luminescens* generally takes 2-3 days, and the factors that control the native *cip* promoters are still unknown. To circumvent these drawbacks, the Cip scaffolds were tested in *Escherichia coli.* First, *cipA-his* and *cipB*-*his* were expressed in *Escherichia coli* DH5α under the control of the arabinose inducible promoter P_{BAD}. When cells were grown at 37°C, PCIs with diameters of 0.1-0.3 µm were detected by phase-contrast microscopy after induction of fusion gene expression with L-arabinose for 2 h (Figure 2A). This confirmed that PCIs can be produced heterologously in *Escherichia coli.*

GFP produced in *Escherichia coli* is normally homogenously distributed (Figure 2B). It was tested whether the Cip scaffolds can subcellularly organize GFP, and produced CipA-GFP and CipB-GFP at different growth temperatures in *Escherichia coli.* Cells grown at 37°C had highly refractile, but weakly fluorescent PCIs (Figure 2B). When cells were grown at 30°C, phase-bright and fluorescent CipA-GFP or CipB-GFP PCIs were detectable (Figure 2B). A further decrease in the growth temperature to 20°C permitted subcellular organization of CipA-GFP and CipB-GFP hybrids to be visualized as highly fluorescent areas, which were not detectable in phase-contrast micrographs (Figure 2B). Furthermore, when the development of CipA-GFP and CipB-GFP protein organization using time-lapse microscopy was analyzed, it was found that the fluorescent complexes did not disassemble during cell growth and cell division (Supporting information, video 1 and video 2).

These results suggest that the protein complexes formed at 30°C/37°C are densely organized and therefore shift the phase of incident light, whereas the complexes produced at 20°C seem to be less tightly packed. The low fluorescence of PCIs produced at 37°C is likely to be the result of rapid assembly of the CipA-GFP or Cip-GFP molecules, which might exceed the maturation rate of the chromophores.

In addition to the temperature, the effect of inducer concentration on Cip scaffold protein organization was also tested. When the L-arabinose concentration was reduced to 0.05% (w/v) or 0.01% (w/v), the PCIs formed by CipA-GFP were quite smaller, but their fluorescence intensity was not affected (Figure 7). This result implies that the inducer concentration has an impact on the rate of synthesis of CipA-GFP molecules, but - unlike temperature - has no influence on the organization of the hybrid protein.

Since low-cost defined media are more commonly used in biotechnological productions, *Escherichia coli* cells were also cultured in defined medium, such as M9-medium, and it was found that CipA-GFP and CipB-GFP assembled into fluorescent PCIs that were clearly visible under the microscope (data not shown).

In summary, Cip scaffolds can organize GFP into subcellular structures in *Escherichia coli.* This process does not require any exogenous co-factors, but is affected by growth temperature. Moreover, the PCIs are very stable *in vivo.* After 6 days of cultivation, the PCIs were still visible in the cells (Figure 8).

### Example 3- Isolation and characterization of CipA-GFP and CipB-GFP PCIs from Escherichia coli

To isolate CipA-GFP and CipB-GFP PCIs from *Escherichia coli,* cells were passed three times through a French press at 0.4 kbar. Then the PCIs were collected by centrifugation and washed three times (Figure 3A). Without any further purification, SDS-PAGE indicated that the isolated PCIs were already about 90% pure (Figure 9). CipA-GFP and CipB-GFP protein complexes produced at 20°C could not be isolated by this procedure as they broke up during cell disruption and could not be pelleted by centrifugation (Figure 9).

To study their thermal stability, the isolated PCIs were exposed to different temperatures for 10 min. It was found that the fluorescence signals emitted by CipA-GFP and CipB-GFP PCIs vanished at 80°C, but PCIs remained structurally undisturbed even after 10 min at 100°C (Figure 3B). Thus, high temperature affects the activity of the tagged protein or enzyme but does not influence PCI structure *per se.*

To determine their solubility, CipA-GFP and CipB-GFP PCIs were resuspended in different solutions. The initial optical density (OD₆₀₀) was measured and any subsequent decrease was taken to reflect solubilization of PCIs (Figure 3C). It was found that PCIs could be dissolved in denaturing solutions such as 10% (w/v) SDS or 8 M urea, and under acidic (pH 3) or alkaline (pH 11) conditions, but also (partially) in 50% (v/v) glycerol. In contrast, high concentrations of NaCl or EDTA had no effect (Figure 3C). In fact, PCIs organized by Cip scaffolds, like many other crystals produced *in vivo,* differ markedly in some physical characteristics from crystals produced *in vitro.* For example, the crystals of *Trypanosoma brucei* cathepsin produced in Sf9 insect cells have been reported to be stable in deionized water, salt solutions or alkaline buffers, but soluble in buffers at pH below 4.*²³* Bt crystals also remain stable in mildly acidic conditions, but can be dissolved at alkaline pH.*²⁴* In summary, our data clearly show that the Cip-based PCIs produced *in vivo* are very stable. Easy isolation of stable proteins is an essential prerequisite for most biotechnological applications *in vitro.*

### Example 4 - Cip scaffolds organize LacZ into enzymatically active PCIs

In the next step, the β-galactosidase (LacZ) derived from *Escherichia coli* was tagged separately with CipA and CipB and produced these hybrid proteins in *Escherichia coli* DH5α at 37°C. It was observed that CipA-LacZ and CipB-LacZ were organized into refractile PCIs (Figure 4A), which could be isolated as easily as the CipA-GFP and CipB-GFP PCls. In parallel, soluble 6His-tagged LacZ (His-LacZ) was purified by affinity chromatography. Then enzymatic activities were determined. Yields of CipA-LacZ and CipB-LacZ were higher relative to that of the soluble His-LacZ (Figure 4B). Conversely, the specific activity of the soluble His-LacZ was higher than that of Cip-tagged LacZ. This might indicate that only molecules on the surface of PCIs are accessible to the substrate *o-*nitrophenyl-β-D-galactopyranoside.

The optimum temperature for His-LacZ activity was determined to be 42°C, while the activities of CipA-LacZ and CipB-LacZ PCIs peaked at 52°C (Figure 4C, left). The pH profiles were similar for LacZ in soluble and PCI forms, all showing an optimal activity at pH 8.0 (Figure 4C, right). This demonstrates that Cip scaffolds are useful tools with which to stabilize enzymes. Furthermore, Cip scaffolds can functionally organize proteins of large size (LacZ = 116 kDa) into PCIs.

### Example 5 - Cip scaffolds co-organize GFP and LacZ into bioactive PCIs

Most metabolic pathways employ multiple enzymes to transform substrates into products. It was therefore asked whether, in addition to organizing single types of proteins, Cip scaffolds could also co-organize multiple proteins. For this purpose, GFP and LacZ (two reporter proteins that share no sequence or structural similarity and differ in molecular weight) were tagged separately with CipA and CipB. CipA-GFP and CipA-LacZ or CipB-GFP and CipB-LacZ were produced in combination in *Escherichia coli* and the PCIs were isolated as described above and analyzed by microscopy. It was found that each of the isolated PCI particles was fluorescent (Figure 5A), indicating that GFP molecules had been incorporated into all PCIs. The isolated PCIs were also analyzed by SDS-PAGE, which clearly revealed two major bands corresponding to CipA-LacZ (or CipB-LacZ) and CipA-GFP (or CipB-GFP) in similar molar ratios (Figure 5B). Moreover, the isolated PCIs also had similar β-galactosidase activities (Figure 5C). Thus, it was concluded that Cip scaffolds can organize GFP and LacZ proteins into mixed PCls, which are both fluorescent and enzymatically active. This suggested that Cip scaffolds might also be capable of organizing multiple enzymes into metabolic pathways.

### Example 6 - The CipA scaffold brings together multiple enzymes involved in violacein biosynthetic pathway

To prove that Cip scaffolds can be used to organize multiple enzymes required for successive steps in a biosynthetic pathway and thus to enhance metabolite production, the enzymes of the violacein biosynthetic pathway were tagged. Violacein is a bisindole compound that is naturally produced by several Gram-negative bacteria i.e. *Chromobacterium violaceum, Janthinobacterium lividum* and *Pseudoalteromonas tunicata.* Due to its potential applications in medicine, its complex synthetic pathway has been well studied.*^{25, 26}* Violacein synthesis starts from L-tryptophan, and involves the five enzymes VioA, VioB, VioE, VioC, VioD (Vio enzymes) (Figure 6A). Two intermediates in the pathway, protodeoxyviolaceinic acid (PDVA) and protoviolaceinic acid (PVA) have been reported to be highly unstable (Figure 6A, underlined).*²⁷* The former is the common precursor of violacein and deoxyviolacein. Deoxyviolacein is an unwanted product, as its formation reduces the amount of protodeoxyviolaceinic acid available for synthesis of violacein (Figure 6A). Organization of the Vio enzymes with the aid of a CipA-based scaffold should bring the Vio enzymes close together in space, perhaps promoting inter-enzyme diffusion and facilitating rapid conversion of unstable metabolites.

A series of production strains, designated *Escherichia coli* Vio1-Vio6, were generated in which none (Vio1), all (Vio6) or some (Vio2, Vio3, Vio4, Vio5) of the five enzymes in the pathway were individually fused to CipA (Figure 10A). The corresponding genes were placed under the control of the P_{BAD} promoter. First, the best inducer concentration for violacein production was determined (Figure 10B), and 0.05% (w/v) L-arabinose was found to be optimal. Cultivation of the respective *Escherichia coli* strains was carried out in LB medium at 30°C for 32 h. The incorporation of Vio enzymes into PCIs in strains *Escherichia coli* Vio2-Vio6 was confirmed (Figure 10C), but the levels of pigment accumulated varied from strain to strain (based on visual inspection, data not shown).

To determine the metabolic flux from the substrate L-tryptophan to the terminal products in more detail, violacein + deoxyviolacein were extracted (as a mixture) with methanol and their relative concentrations were quantified during growth of the engineered strains (Figure 6B). The final yields of violacein + deoxyviolacein were 73.8 µM (Vio1: all Vio enzymes free), 51.5 µM [Vio2: CipA-(VioA,VioB), all other enzymes free] 51.5 µM [Vio3: CipA-(VioA,VioB,VioD,VioE); VioC free], 272.4 µM [Vio4: CipA-(VioD,VioE), others free], 210.6 µM [Vio5: CipA-(VioC,VioD,VioE); VioA and VioB free] and 10.9 µM (Vio6: CipA-all Vio enzymes) (Figure 6B). These results show that productivity is very much improved in *Escherichia coli* strains Vio4 and Vio5 in comparison to Vio1. While the maximum rate of synthesis of violacein + deoxyviolacein for *Escherichia coli* Vio1 was 2.1 µM/h, it was 16.4-fold higher (36.5 µM/h) and 8.5-fold higher (20.0 µM/h) in strains *Escherichia coli* Vio4 and *Escherichia coli* Vio5, respectively.

In the next step the ratios of violacein to deoxyviolacein were determined (Figure 6B). *Escherichia coli* strains Vio3 and Vio6 had the lowest level of the by-product, but the overall yield was much lower than in *Escherichia coli* strain Vio4. Overall, however, *Escherichia coli* Vio5 was characterized to show the highest production of violacein, which was determined to be 183.2 µM. In this strain VioC, VioD and VioE were organized into PCls, while VioA and VioB were synthesized as free enzymes. Since VioE and VioD are, respectively, responsible for synthesis of the unstable intermediates PDVA and PVA, while VioC converts these into deoxyviolacein and violacein, respectively, encapsulation of the three enzymes clearly enhances both the overall productivity of the pathway - and selectivity for violacein.

In summary, two small proteins CipA and CipB derived from *Photorhabdus luminescens* can function as Cip scaffolds to organize proteins into PCIs in both the native host or heterologeously in *Escherichia coli* (Figures 1 and 2). Stable protein organization is beneficial for both *in vitro* and *in vivo* applications (Figures 5 and 6). For *in vitro* applications, the ability to organize proteins into PCIs via Cip scaffolds is a significant advantage, since PCIs are resistant to cell disruption and can be isolated in high yield and purity within less than 2 h (Figure 3). Furthermore, enzymes, such as β-galactosidase, were more heat stable when incorporated into PCIs (Figure 4C). For *in vivo* applications, the use of Cip scaffolds enables flexible organization of enzymes into complexes and soluble forms, which can help to improve the overall yield of the desired product, and reduce side-product formation, as shown here for violacein biosynthesis (Figure 6).

### Example 7- Methods

### Strains and growth conditions

The bacterial strains used in this study are listed in Figure 12A. All *Escherichia coli* strains were aerobically cultivated in Lysogeny Broth (LB)*²⁸* or on LB agar at the indicated temperatures. When required, the medium was supplemented with 100 µL/mL ampicillin and/or 15 µg/mL chloramphenicol. For induction of arabinose promoter (P_{BAD}) controlled gene expression, 0.2% (w/v) L-arabinose was added to cultures when the OD₆₀₀ reached 0.6. For growth of *Escherichia coli* ST18*²⁹*, media were supplemented with 5-aminolevulinic acid (50 µg/mL). *Photorhabdus luminescens* was grown in casein-soya-peptone (CASO) medium at 30°C.*³⁰* When necessary, the medium was supplemented with 25 µg/mL of kanamycin. *Chromobacterium violaceum* DSM 30191 was grown in LB medium at 30°C.

### Plasmid construction

All plasmids used in this study are listed in Figure 12A, and the primers used for PCR amplification of the DNA fragments needed to assemble these plasmids are listed in Figure 12B. The gene fusions were created with two-step PCR. All restriction digests of PCR-amplified DNA fragments and pBAD18 or pBAD33 vectors were performed with the enzymes indicated in the names of the primers used for PCR experiments.

To generate plasmid pBAD-cipA-6His, pBAD-cipB-6His, pBAD-cipA-gfp, pBAD-cipB-gfp, pBAD-cipA-lacZ, and pBAD-cipB-lacZ, the respective genes or gene fusions were amplified using chromosomal DNA of *Photorhabdus luminescens* or *Escherichia coli* as template. The DNA fragments were then digested and ligated into the correspondingly resected vector pBAD18.*³¹* Plasmids pBAD-cipA-(gfp,lacZ) and pBAD-cipB-(gfp,lacZ) were generated by successively ligating *cipA-gfp* and *cipA-lacZ* or *cipB-gfp* and *cipB-lacZ* into pBAD18. To generate the pBAD-free-(vioA,vioB) and pBAD-cipA-(vioA,vioB) constructs, *vioA* and *vioB* genes or *cipA-vioA* and *cipA-vioB* fusions were amplified from chromosomal DNA of *Photorhabdus luminescens* or *Chromobacterium violaceum* DSM 30191. DNA fragments were digested and then ligated successively into the appropriately digested pBAD18. In the same way, pBAD-free-(vioC,vioD,vioE), pBAD-free-(vioC)-cipA-(vioD,vioE) and pBAD-cipA-(vioC,vioD,vioE) plasmids were constructed by successively ligating *vioC, vioD* and *vioE* genes, either alone or as *cipA-vioC, cipA-vioD, cipA-vioE* fusions, into the pBAD33 vector (pBAD18 compatible).*³¹* All genes or gene fusions were ligated into vectors in such a way that they can be expressed by induction of the arabinose-dependent P_{BAD} promoter encoded on the vector backbone, but retain their own Shine-Dalgarno sequence and stop codon. Note that, in every case where a protein was tagged with a Cip scaffold, no intervening linker of any kind was used.

### Generation of the strains Escherichia coli Vio1 to Vio6

Violacein production strains *Escherichia coli* Vio1 to Vio6 were generated by co-transformation of *Escherichia coli* DH5α with the respective pBAD18 and pBAD33 derivatives containing free or *cipA-*fused *vio* genes as listed in Figure 12A and depicted in Figure 10A. For use as a negative control strain for violacein production, *Escherichia coli* Vio0 was obtained by co-transformation of *Escherichia coli* DH5α with the empty vectors pBAD18 and pBAD33.

### Generation of Photorhabdus luminescens ΔcipA and/or ΔcipB and CipA-GFP or CipB-GFP production strains

To generate a clean deletion of *cipA* or *cipB* in *Photorhabdus luminescens,* two 700-bp fragments homologous to the upstream and downstream regions of the *cipA* and *cipB* genes, respectively, were amplified using *Photorhabdus luminescens* chromosomal DNA and the appropriate primers listed in Figure 12B. The resulting products were then combined into one DNA fragment via a second PCR step, and inserted into the suicide vector pNPTS-138-R6KT*³²* to obtain pNPTS-138-R6KT-del-cipA and pNPTS-138-R6KT-del-cipB, respectively. *Escherichia coli* ST18*²⁹* transformed with these plasmids was used for conjugation of *Photorhabdus luminescens* TT01, and chromosomal integrants were selected on the basis of their kanamycin resistance.*³³* These cells were grown in CASO medium and then plated on CASO agar containing 10% (w/v) sucrose in the absence of kanamycin to select for segregation of the plasmid backbone from the chromosome. Single colonies were tested for deletion of *cipA* or *cipB* and loss of the marker gene *sacB,* which confers sucrose sensitivity.*³⁴ Photorhabdus luminescens* TT01 *ΔcipA*/*ΔcipB* was generated by successive deletion of *cipA* and *cipB,* and each recombination step was confirmed by colony PCR. To generate *Photorhabdus luminescens* cells that produce CipA-GFP or CipB-GFP, *P_{cipA}-cipA-gfp* or *P_{cipB}-cipB-gfp* (synthesized by overlap PCR; *P_{cipA}* or *P_{cipB}* stands for the native promoters of *cipA* and *cipB*) was ligated into the pPINT vector*³³* to create pPINT-P_{cipA}-cipA-gfp and pPINT-P_{cipB}-cipB-gfp. These two plasmids were separately conjugated from *Escherichia coli* ST18 into *Photorhabdus luminescens* TT01 *ΔcipA*/*ΔcipB* and integrated into the genome as described.*³³* Recombination events were verified by colony PCR.

### Isolation and characterization of PCIs

To isolate PCls, cells were collected by centrifugation, resuspended in ice-cold sodium-phosphate buffer (20 mM sodium phosphate buffer, pH 7.0, 8.5 mM KCI), and disrupted by three passages through a French press (Thermo IEC Laboratory Press) at 0.4 kbar. PCIs were collected by centrifugation for 15 min at 4.000 x g, and washed three times with the same buffer.

The protein content of PCI samples was determined by measuring band intensities after SDS-PAGE.*³⁵* Gels were stained with Coomassie Brilliant Blue R-250 and band intensities were calculated with **ImageQuant** TL using 0, 0.4 µg, 1 µg, 2 µg, 3 µg, 4 ug and 5 µg of BSA for calibration (Figure 11).

Thermal stability studies were performed in sodium-phosphate buffer. The isolated CipA-GFP and CipB-GFP PCIs were incubated for 10 min at 30°C, 40°C, 50°C, 60°C, 70°C, 80°C, 90°C and 100°C, respectively, and PCI shape and fluorescence intensity were subsequently determined by microscopy.

To test the solubility of PCls, CipA-GFP and CipB-GFP PCIs were resuspended at an OD₆₀₀ of 20-60 in deionized water. Then, 10 µL of this suspension was added to, and gently mixed with 1 mL of the following solutions: (i) deionized water; (ii) 150 mM NaCl, pH 1.0, 3.0, 5.0, 7.0, 9.0, 11.0 or 13.0; (iii) 1 M, 2 M, 3 M, and 4 M NaCl, pH 7.0; (iv) 2 M, 4 M, 6 M, 8 M urea; (v) 100 mM EDTA, pH 7.0; (vi) 10% (w/v) SDS, pH 7.0; (vii) 100% (v/v) methanol, and (viii) 50% (v/v) glycerol, respectively. The OD₆₀₀ was then monitored periodically and set in relation to that of the corresponding control (PCIs in deionized water), which was set to 100%.

### Microscopy

Phase-contrast as well as fluorescence microscopy was performed with a Leica DMI6000 B microscope, and the pictures were taken and evaluated with the Leica LAS X and LAS AF software. The glass slides were previously covered with agarose in 0.9% (w/v) NaCI solution to create a flat, thin layer pad (less than 1 mm thick). Then, 3 µL of fresh culture or isolated PCI suspension was loaded onto the agarose pads. For time-lapse microscopy, a glass-bottomed dish with a well (In Vitro Scientific) was covered with 0.5%-0.8% (w/v) agarose in LB medium supplemented with 100 µg/mL ampicillin and 0.2% (w/v) arabinose. Fresh cultures in the early exponential phase (OD₆₀₀=0.6) were diluted to OD₆₀₀=0.1 with LB medium and inoculated onto the LB-agar pad, which was subsequently flipped. The dish was covered and incubated at 20°C under the microscope, and phase-contrast as well as fluorescence micrographs were taken every 10 min. Videos were then created from these images using Fiji-lmageJ software.*³⁶*

### Overproduction, purification and assay of β-galactosidase (LacZ)

For the production and isolation of CipA-LacZ or CipB-LacZ PCls, procedure described above were followed. To purify LacZ in a soluble state, N-terminally 6His-tagged LacZ (His-LacZ) was produced in *Escherichia coli* BL21. An overnight culture (20 ml) of cells carrying pBAD-6his-lacZ was used to inoculate two 2-I flasks, each containing 500 ml of LB medium supplemented with 100 µL/mL ampicillin. When the cell density reached OD₆₀₀ 0.8, 0.3% (w/v) L-arabinose was added to induce gene expression, and the cultures were further incubated at 37°C for 3 h. Cells were then harvested by centrifugation, suspended in lysis buffer (50 mM Tris-HCl pH 7.0, 300 mM NaCl, 10 mM β-mercaptoethanol, 10 mM imidazole, 10 mM MgCl₂, Sigma-Aldrich EDTA-free protease inhibitor cocktail), and disrupted in a French press. Cell debris was removed by centrifugation for 20 min at 14,500 x g, and the supernatant was loaded onto 4 ml of nickel-charged resin previously equilibrated with binding buffer (50 mM Tris-HCl, pH 7.0, 300 mM NaCl, 10 mM β-mercaptoethanol, 10 mM imidazole, 20 mM MgCl₂). Columns were washed with 70 ml of washing buffer (binding buffer containing 20 mM imidazole), and His-LacZ was eluted with 17 ml of elution buffer (binding buffer containing 200 mM imidazole). Imidazole was later removed by dialysis of the protein against buffer (20 mM Tris-HCl, pH 7.0, 150 mM NaCl, 10 mM β-mercaptoethanol, 5 mM MgCl₂).

β-Galactosidase activity assays were performed at 30°C in modified buffer Z and quantified as described before.³⁷ One unit equals the amount of enzyme needed to hydrolyze 2-nitrophenyl β-D-galactopyranoside (oNPG) to 1 µmol of o-nitrophenol in 1 min at 30°C. The protein concentration was determined with SDS-PAGE and ImageQuant TL software as described above. Specific enzyme activity was defined as the activity present in 1 mg of His-LacZ, or CipA-GFP or CipB-GFP PCIs.

To test the influence of pH on the activity of His-LacZ, CipA-LacZ PCIs and CipB-LacZ PCIs, the pH of modified buffer Z was adjusted to 5.0, 6.0, 7.0, 8.0, 9.0, and 10.0, and β-galactosidase activity assays were performed at 30°C as described above. The enzyme activities measured at the different pH values were expressed relative to the activity measured at pH 7.0. To determine temperature dependence, the reaction temperature was adjusted to 22°C, 30°C, 37°C, 42°C, 47°C, 52°C, 57°C and 62°C, and measured activities were expressed relative to that at 30°C.

### Violacein/deoxyviolacein production and quantification

To determine the optimal concentration of inducer for violacein production, cultures of the engineered strains (*Escherichia coli* Vio1 to *Escherichia coli* Vio6) in early exponential phase were diluted with LB to an OD₆₀₀ of 0.05 and drops were placed on LB plates containing 0%, 0.01%, 0.02%, 0.05%, 0.1% and 0.4% (w/v) of L-arabinose. After overnight cultivation at 30°C, the best inducer concentration was selected based on pigment accumulation (Figure 10A).

To quantify violacein/desoxyviolacein production *Escherichia coli* strains Vio1-Vio6 were aerobically incubated in LB medium, supplemented with 100 µg/mL ampicillin, 15 µg/mL chloramphenicol and 0.05% (w/v) L-arabinose, at 30°C. Aliquots (1 ml) were taken at the indicated time points, centrifuged, and the pellets were resuspended in 1 ml of 95% (v/v) methanol, vortexed, and cell debris was removed by centrifugation. The concentration of the violacein + deoxyviolacein mixture was determined based on absorbance at A₅₇₇ₙₘ, based on a molar extinction coefficient of 1.7×10⁴ L mol⁻¹ cm⁻¹.*³⁸* To determine the proportions of violacein and deoxyviolacein in the methanol extracts, a HPLC protocol was used as described,*³⁹* but with modifications for use with the Hypersil GOLD aQ column (Thermo Scientific). Briefly, two solvents were used to perform gradient elution. Solvent A was 0.1% (v/v) formic acid in water; solvent B was 0.1% (v/v) formic acid in acetonitrile. The column was equilibrated with 95% (v/v) A + 5% (v/v) B for 6 min; 50 µL methanol extract was then injected; the solvent gradient was started 7 min after injection, and over a period of 10 min, B was increased to 98%; the 2% (v/v) A + 98% (v/v) B mixture was kept constant for 7 min and then replaced by 95% (v/v) A + 5% (v/v) B for 2 min. Light absorbance of the peaks was analyzed with a UV/VIS detector at a wavelength of 567 nm.*³⁹*

### REFERENCES

[1] Shajani, Z., Sykes, M. T., and Williamson, J. R. (2011) Assembly of bacterial ribosomes, Annu. Rev. Biochem. 80, 501-526.
[2] Nickelsen, J., and Rengstl, B. (2013) Photosystem II assembly: from cyanobacteria to plants, Annu. Rev. Plant Biol. 64, 609-635.
[3] Yeates, T. O., Kerfeld, C. A., Heinhorst, S., Cannon, G. C., and Shively, J. M. (2008) Protein-based organelles in bacteria: carboxysomes and related microcompartments, Nat. Rev. Microbiol. 6, 681-691.
[4] Kerfeld, C. A., and Melnicki, M. R. (2016) Assembly, function and evolution of cyanobacterial carboxysomes, Curr. Opin. Plant Biol. 31, 66-75.
[5] Pfeifer, F. (2012) Distribution, formation and regulation of gas vesicles, Nat. Rev. Microbiol. 10, 705-715.
[6] Agaisse, H., and Lereclus, D. (1995) How does Bacillus thuringiensis produce so much insecticidal crystal protein?, J. Bacteriol. 177, 6027-6032.
[7] Schnepf, E., Crickmore, N., Van Rie, J., Lereclus, D., Baum, J., Feitelson, J., Zeigler, D. R., and Dean, D. H. (1998) Bacillus thuringiensis and its pesticidal crystal proteins, Microbiol. Mol. Biol. Rev. 62, 775-806.
[8] Pond, F. R., Gibson, I., Lalucat, J., and Quackenbush, R. L. (1989) R-body-producing bacteria, Microbiol. Rev. 53, 25-67.
[9] Polka, J. K., and Silver, P. A. (2016) A Tunable Protein Piston That Breaks Membranes to Release Encapsulated Cargo, ACS Synth Biol 5, 303-311.
[10] Whitaker, W. R., and Dueber, J. E. (2011) Metabolic pathway flux enhancement by synthetic protein scaffolding, Methods Enzymol. 497, 447-468.
[11] Lee, H., DeLoache, W. C., and Dueber, J. E. (2012) Spatial organization of enzymes for metabolic engineering, Metab Eng 14, 242-251.
[12] Kim, E. Y., and Tullman-Ercek, D. (2013) Engineering nanoscale protein compartments for synthetic organelles, Curr. Opin. Biotechnol. 24, 627-632.
[13] Huber, M. C., Schreiber, A., von Olshausen, P., Varga, B. R., Kretz, O., Joch, B., Barnert, S., Schubert, R., Eimer, S., Kele, P., and Schiller, S. M. (2015) Designer amphiphilic proteins as building blocks for the intracellular formation of organelle-like compartments, Nat Mater 14, 125-132.
[14] Dueber, J. E., Wu, G. C., Malmirchegini, G. R., Moon, T. S., Petzold, C. J., Ullal, A. V., Prather, K. L., and Keasling, J. D. (2009) Synthetic protein scaffolds provide modular control over metabolic flux, Nat. Biotechnol. 27, 753-759.
[15] Myhrvold, C., Polka, J. K., and Silver, P. A. (2016) Synthetic Lipid-Containing Scaffolds Enhance Production by Colocalizing Enzymes, ACS Synth Biol.(in press)
[16] Park, M., Sun, Q., Liu, F., DeLisa, M. P., and Chen, W. (2014) Positional assembly of enzymes on bacterial outer membrane vesicles for cascade reactions, PLoS One 9, e97103.
[17] Lawrence, A. D., Frank, S., Newnham, S., Lee, M. J., Brown, I. R., Xue, W. F., Rowe, M. L., Mulvihill, D. P., Prentice, M. B., Howard, M. J., and Warren, M. J. (2014) Solution structure of a bacterial microcompartment targeting peptide and its application in the construction of an ethanol bioreactor, ACS Synth Biol 3, 454-465.
[18] Gao, X., Yang, S., Zhao, C., Ren, Y., and Wei, D. (2014) Artificial multienzyme supramolecular device: highly ordered self-assembly of oligomeric enzymes in vitro and in vivo, Angew. Chem. Int. Ed. Engl. 53, 14027-14030.
[19] Bintrim, S. B., and Ensign, J. C. (1998) Insertional inactivation of genes encoding the crystalline inclusion proteins of Photorhabdus luminescens results in mutants with pleiotropic phenotypes, J. Bacteriol. 180, 1261-1269.
[20] Bowen, D. J., and Ensign, J. C. (2001) Isolation and characterization of intracellular protein inclusions produced by the entomopathogenic bacterium Photorhabdus luminescens, Appl. Environ. Microbiol. 67, 4834-4841.
[21] You, J., Liang, S., Cao, L., Liu, X., and Han, R. (2006) Nutritive significance of crystalline inclusion proteins of Photorhabdus luminescens in Steinernema nematodes, FEMS Microbiol. Ecol. 55, 178-185.
[22] Goodrich-Blair, H., and Clarke, D. J. (2007) Mutualism and pathogenesis in Xenorhabdus and Photorhabdus: two roads to the same destination, Mol. Microbiol. 64, 260-268.
[23] Koopmann, R., Cupelli, K., Redecke, L., Nass, K., Deponte, D. P., White, T. A., Stellato, F., Rehders, D., Liang, M., Andreasson, J., Aquila, A., Bajt, S., Barthelmess, M., Barty, A., Bogan, M. J., Bostedt, C., Boutet, S., Bozek, J. D., Caleman, C., Coppola, N., Davidsson, J., Doak, R. B., Ekeberg, T., Epp, S. W., Erk, B., Fleckenstein, H., Foucar, L., Graafsma, H., Gumprecht, L., Hajdu, J., Hampton, C. Y., Hartmann, A., Hartmann, R., Hauser, G., Hirsemann, H., Holl, P., Hunter, M. S., Kassemeyer, S., Kirian, R. A., Lomb, L., Maia, F. R., Kimmel, N., Martin, A. V., Messerschmidt, M., Reich, C., Rolles, D., Rudek, B., Rudenko, A., Schlichting, I., Schulz, J., Seibert, M. M., Shoeman, R. L., Sierra, R. G., Soltau, H., Stern, S., Struder, L., Timneanu, N., Ullrich, J., Wang, X., Weidenspointner, G., Weierstall, U., Williams, G. J., Wunderer, C. B., Fromme, P., Spence, J. C., Stehle, T., Chapman, H. N., Betzel, C., and Duszenko, M. (2012) In vivo protein crystallization opens new routes in structural biology, Nat Methods 9, 259-262.
[24] Sawaya, M. R., Cascio, D., Gingery, M., Rodriguez, J., Goldschmidt, L., Colletier, J. P., Messerschmidt, M. M., Boutet, S., Koglin, J. E., Williams, G. J., Brewster, A. S., Nass, K., Hattne, J., Botha, S., Doak, R. B., Shoeman, R. L., DePonte, D. P., Park, H. W., Federici, B. A., Sauter, N. K., Schlichting, I., and Eisenberg, D. S. (2014) Protein crystal structure obtained at 2.9 A resolution from injecting bacterial cells into an X-ray free-electron laser beam, Proc. Natl. Acad. Sci. U. S. A. 111, 12769-12774.
[25] Hoshino, T. (2011) Violacein and related tryptophan metabolites produced by Chromobacterium violaceum: biosynthetic mechanism and pathway for construction of violacein core, Appl. Microbiol. Biotechnol. 91, 1463-1475.
[26] Sanchez, C., Brana, A. F., Mendez, C., and Salas, J. A. (2006) Reevaluation of the violacein biosynthetic pathway and its relationship to indolocarbazole biosynthesis, Chembiochem 7, 1231-1240.
[27] Shinoda, K., Hasegawa, T., Sato, H., Shinozaki, M., Kuramoto, H., Takamiya, Y., Sato, T., Nikaidou, N., Watanabe, T., and Hoshino, T. (2007) Biosynthesis of violacein: a genuine intermediate, protoviolaceinic acid, produced by VioABDE, and insight into VioC function, Chem. Commun. (Camb.) 40, 4140-4142.
[28] Bertani, G. (1951) Studies on lysogenesis. I. The mode of phage liberation by lysogenic Escherichia coli, J. Bacteriol. 62, 293-300.
[29] Thoma, S., and Schobert, M. (2009) An improved Escherichia coli donor strain for diparental mating, FEMS Microbiol. Lett. 294, 127-132.
[30] Munch, A., Stingl, L., Jung, K., and Heermann, R. (2008) Photorhabdus luminescens genes induced upon insect infection, BMC Genomics 9, 229.
[31] Guzman, L. M., Belin, D., Carson, M. J., and Beckwith, J. (1995) Tight regulation, modulation, and high-level expression by vectors containing the arabinose PBAD promoter, J. Bacteriol. 177, 4121-4130.
[32] Lassak, J., Henche, A. L., Binnenkade, L., and Thormann, K. M. (2010) ArcS, the cognate sensor kinase in an atypical Arc system of Shewanella oneidensis MR-1, Appl. Environ. Microbiol. 76, 3263-3274.
[33] Glaeser, A., and Heermann, R. (2015) A novel tool for stable genomic reporter gene integration to analyze heterogeneity in Photorhabdus luminescens at the single-cell level, Biotechniques 59, 74-81.
[34] Blomfield, I. C., Vaughn, V., Rest, R. F., and Eisenstein, B. I. (1991) Allelic exchange in Escherichia coli using the Bacillus subtilis sacB gene and a temperature-sensitive pSC101 replicon, Mol. Microbiol. 5, 1447-1457.
[35] Laemmli, U. K. (1970) Cleavage of structural proteins during the assembly of the head of bacteriophage T4, Nature 227, 680-685.
[36] Schindelin, J., Arganda-Carreras, I., Frise, E., Kaynig, V., Longair, M., Pietzsch, T., Preibisch, S., Rueden, C., Saalfeld, S., Schmid, B., Tinevez, J. Y., White, D. J., Hartenstein, V., Eliceiri, K., Tomancak, P., and Cardona, A. (2012) Fiji: an open-source platform for biological-image analysis, Nat Methods 9, 676-682.
[37] Miller, J. H. (1972) Experiments in molecular genetics, Cold Spring Harbor Laboratory, Cold Spring Harbor, N.Y.
[38] Sneath, P. H. (1956) Cultural and biochemical characteristics of the genus Chromobacterium, J. Gen. Microbiol. 15, 70-98.
[39] Lee, M. E., Aswani, A., Han, A. S., Tomlin, C. J., and Dueber, J. E. (2013) Expression-level optimization of a multi-enzyme pathway in the absence of a high-throughput assay, Nucleic Acids Res. 41, 10668-10678.

## Claims

1. A method for producing at least two proteins in the form of protein crystalline inclusions (PCIs) in a bacterial host cell, wherein the method comprises
(a) introducing one or more fusion constructs into the bacterial host cell, said one or more fusion constructs comprising in expressible form one or more nucleic acid molecules encoding the at least two proteins, wherein each of the one or more nucleic acid molecules is independently fused to a nucleic acid molecule being selected from the group consisting of the nucleic acid molecules having
i. the nucleotide sequence of SEQ ID NO: 1,
ii. the nucleotide sequence of SEQ ID NO: 2,
iii. the nucleotide sequence of SEQ ID NO: 3,
iv. a nucleotide sequence being at least 80% identical to SEQ ID NO: 1, wherein the encoded protein retains the capability of forming PCIs,
v. a nucleotide sequence being at least 80% identical to SEQ ID NO: 2, wherein the encoded protein retains the capability of forming PCIs,
vi. a nucleotide sequence being at least 80% identical to SEQ ID NO: 3, wherein the encoded protein retains the capability of forming PCIs,
vii. a nucleotide sequence encoding the amino acid sequence of SEQ ID NO: 4,
viii. a nucleotide sequence encoding the amino acid sequence of SEQ ID NO: 5,
ix. a nucleotide sequence encoding the amino acid sequence of SEQ ID NO: 6,
x. a nucleotide sequence encoding an amino acid sequence being at least 80% identical to SEQ ID NO: 4, wherein the encoded protein retains the capability of forming PCIs,
xi. a nucleotide sequence encoding an amino acid sequence being at least 80% identical to SEQ ID NO: 5, wherein the encoded protein retains the capability of forming PCIs, and
xii. a nucleotide sequence encoding an amino acid sequence being at least 80% identical to SEQ ID NO: 6, wherein the encoded protein retains the capability of forming PCIs;
and
(b) culturing the bacterial host cell obtained in (a) to produce the at least two proteins in the form of PCIs.

2. The method of claim 1 further comprising purifying the PCIs from the bacterial host cell.

3. The method of claim 1 or 2 further comprising purifying the proteins from the PCIs.

4. The method of any of claims 1 to 3, wherein the at least two proteins are at least two proteins involved in the same multi-step biosynthetic pathway.

5. The method of claim 4 further comprising producing at least one compound being a product of said multi-step biosynthetic pathway, wherein all proteins being required for the production of said at least one compound are contained in said bacterial host cell in expressible form and optionally further comprising purifying the at least one compound from the bacterial host cell.

6. The method of claim 5, wherein the compound is an intermediate or an end product of the multi-step biosynthetic pathway.

7. The method of any one of claims 1 to 6, wherein the proteins are enzymes.

8. The method of claim 7, wherein the enzymes are involved in a biosynthetic pathway to produce a primary or secondary metabolite and/or to increase the yield of such a metabolite.

9. The method of claim 8, wherein the enzymes are selected from VioA, VioB, VioC, VioD and VioE.

10. The method of claim 9, wherein
(i) VioD and VioE are produced in the form of PCIs, and VioA, VioB and VioC are produced in PCI-free form,
(ii) VioC, VioD and VioE are produced in the form of PCIs, and VioA and VioB are produced in PCI-free form, or
(iii) three, and preferably four or all five of VioA, VioB, VioC, VioD and VioE are produced in the form of PCIs.

11. The method of claim 9 or 10, wherein the at least one compound is violacein.

12. The method of any one of claims 1 to 11, wherein the bacterial host is a proteobacterium, preferably a gammaproteobacterium, more preferably an enterobacterium, and is most preferably *Escherichia coli, Photorhabdus spec.,* or *Xenorhabdus spec.,* wherein the genes *cipA* and *cipB* of *Photorhabdus spec.,* and the gene *pixA* of *Xenorhabdus spec.* are inactivated.

13. A fusion construct comprising in expressible form one or more nucleic acid molecules encoding at least two proteins to be produced in the form of PCIs, wherein each of the one or more nucleic acid molecules is independently fused to a nucleic acid molecule being selected from the group of nucleic acid molecules pursuant to items i. to xii. as defined in claim 1.

14. A bacterial host cell comprising one or more fusion constructs as defined in any one of claims 1 to 12.

15. A method for producing at least one protein in the form of PCIs in an *Escherichia coli* host cell, wherein the method comprises
(a) introducing into the *Escherichia coli* host cell a fusion construct, said fusion construct comprising in expressible form a nucleic acid molecule encoding the least at least one protein fused to a nucleic acid molecule being selected from the group of nucleic acid molecules pursuant to items i. to xii. as defined claim 1.
and
(b) culturing the *Escherichia coli* host cell obtained in (a) to produce the at least one heterologous protein in the form of PCIs.
